# EUROPEAN PATENT APPLICATION

(11) **EP 4 663 656 A2**
(43) Date of publication of application: **17.12.2025**
(21) Application number: 25212162.9
(22) Date of filing: 09.03.2023
(51) Int. Cl.: C07K 16/18

(54) **MASP-2 AND MASP-3 INHIBITORS, AND RELATED COMPOSITIONS AND METHODS, FOR TREATMENT OF SICKLE CELL DISEASE**

(30) Priority: 10.03.2022 US 202263318582 P
(62) Divisional of application: 23767710.9
(71) Applicant: Omeros Corporation, Seattle, WA 98119 (US); Regents of the University of Minnesota, Minneapolis, MN 55455-2020 (US)
(72) Inventor: BELCHER, John D, Minneapolis 55455-2020 (US); CUMMINGS, William, Jason, Seattle 98119 (US); DUDLER, Thomas, A., Seattle 98119 (US); VERCELLOTTI, Gregory, M., Minneapolis 55455-2020 (US)
(74) Representative: Avidity IP

(57) **Abstract**

The present disclosure relates to the use of MASP-2 inhibitors and/or MASP-3 inhibitors and compositions comprising the same for treatment of sickle cell disease, including treatment, reduction, and/or prevention of sickle cell disease symptoms or manifestations.

## Description

### CROSS-REFERENCE TO RELATED APPLICATIONS

This application claims the benefit of U.S. Provisional Application No. 63/318,582, filed March 10, 2022, which is incorporated herein by reference in its entirety.

### FEDERALLY SPONSORED RESEARCH

This invention was made with government support under HL114567 awarded by the National Institutes of Health. The government has certain rights in the invention.

### FIELD OF THE INVENTION

The present invention relates to MASP-2 inhibitors and MASP-3 inhibitors and related compositions and methods.

### STATEMENT REGARDING SEQUENCE LISTING

The sequence listing associated with this application is provided in XML format in lieu of a paper copy and is hereby incorporated by reference into the specification. The name of the XML file containing the sequence listing is: MP_1_0313_US2_Sequence Listing_ST26_20230308. The XML file is 32,265 bytes; was created on March 8, 2023, and is being submitted via the Patent Center with the filing of the specification.

### BACKGROUND

Sickle cell disease (SCD) is a painful disorder that afflicts millions of people worldwide. SCD is an autosomal recessive disorder caused by an amino acid substitution in the beta-globin gene, that causes hemoglobin S polymerization and red blood cell (RBC) sickling when deoxygenated. This causes altered RBC physiology, hemolysis, anemia, and recurrent episodes of painful vaso-occlusion. Hebbel et al., J Clin Invest 130:1062 (2020).

Vaso-occlusive pain crisis (VOC) is the leading cause of emergency department visits and hospitalizations in the SCD population. Platt et al., N Engl J Med 325:11 (1991). Vaso-occlusion can be caused by the direct obstruction by sickle erythrocytes in smaller vessels or by the adhesion of sickle erythrocytes and leukocytes to the endothelium of larger vessels.

In SCD patients and mice, complement has been shown to be abnormally activated in steady state, during crisis, and in patients with delayed hemolytic transfusion reactions. The first report of complement activation in SCD was published in 1967, and many studies have since reported increased levels of complement-derived fragments in the blood of SCD patients, demonstrating that complement is activated in SCD and suggesting that complement may play an important role in the pathophysiology of the disease. Francis et al., Am J Med Tech 33:77 (1967); Gavriilaki et al., Haem 102:e481 (2017); Lombardi et al., Haem 104:919 (2019), Merle et al., JCI Insight 3 (2018); Roumenina et al., Am J Hemat 95:456 (2020); Yoo et al., Blood 138:858 (2021). Lombardi and colleagues found increased serum levels of complement anaphylatoxin C5a in SCD patients, deposition of C5b-9 in small vessels of skin biopsies, and C3b on sickle (SS) RBC membranes. These SS RBCs were more adhesive to endothelium with adhesion inhibited by complement factor H, a soluble plasma regulator of the alternative pathway. Roumenina and colleagues showed that treatment of SCD patients with hydroxyurea reduced plasma C5b-9 and C3d deposition on SS RBCs. Yoo et al. reported that Bb, a fragment of factor B and an enzymatic component of the alternative pathway C3 convertase, and anaphylatoxins C3a and C5a were elevated in the plasma of pediatric patients during vaso-occlusive pain crises compared to steady-state. In SS mice, infusion of C5a quickly promoted vaso-occlusion and tissue inflammation and these effects were abrogated by infusing anti-C5a receptor (C5aR) monoclonal antibodies (mAb) that block C5a/C5aR proinflammatory signaling. Vercellotti et al., Am J Hemat 94:327 (2019). However, the mechanisms driving complement activation in SCD have not been completely elucidated.

Heme has been implicated in alternative pathway activation in SCD. Heme released during intravascular hemolysis in SCD activates the alternative pathway on endothelial surfaces and this complement activation is attenuated in vivo and in vitro by the heme scavenger hemopexin. Merle et al., 2018. The alternative pathway C3 and C5 convertases can assemble on the A3 domain of ultralarge von Willebrand factor (Nolasco et al., TH Open 2; e338 (2018)) and by noncovalent anchoring of C3(H2O) and C3 activation fragments to P-selectin expressed on endothelial cells in a toll-like receptor 4 (TLR4)-dependent manner (Merle et al., PNAS 116:6280 (2019)).

In addition to alternative pathway activation by heme, other complement pathways also may be activated in SCD. Ischemia-reperfusion (I/R) and endothelial damage are central to the pathophysiology of SCD. Hebbel et al., J. Clin Invest 130:1062 (2020); Nader et al., Compr Physil 11:1785 (2021); Kato et al., Am J Hemat 84:618 (2009). Endothelial injury can occur as a direct response to I/R and oxidative stress. Sunnergren and Rovetto, Am J Physiol 252, H1211 (1987); Taylor, Proc Natl Sci Counc Repub China 15:191 (1991) Aki et al., Brain Res 1292:180 (2009). Endothelial injury can trigger activation of the lectin pathway (LP), promoting inflammation and further endothelial injury. Gavriilaki et al., Exp Hematol Oncol 10:57 (2021); Defendi et al., Front Immunol 12:741446 (2021); Ali et al., Front Immunol 12:714511 (2021); Rambaldi et al., Immunobiol 225:152001 (2020). Activation of the LP may also trigger coagulation via MASP-2 cleavage of prothrombin to thrombin. Kozarcanin et al., J Throm Haemo 14:531 (2016); Gulla et al., Immunol 129:482 (2010). In experimental I/R, inhibition of the LP blocks subsequent inflammatory responses and organ damage. Farrar et al., Front Immunol 3:341 (2012); de Vries et al., Am J Path 165:1677 (2004); Jordan et al., Circul 104:1413 (2001); La Bonte et al., Diab & Vasc Dis Res 6:172 (2009); Orsini et al., J Neuroinflam 13:213 (2016); Panagiotou et al., Front Immunol 9:1151 (2018); Sandgaard et al., Mol Neurobio 56:78 (2019); Schwaeble et al., PNAS USA 108:7523 (2011). Experimental I/R in SS mice can be achieved using hypoxia-reoxygenation, which activates complement and induces vascular inflammation and vaso-occlusion that are inhibited by infusing anti-C5aR or anti-C5 monoclonal antibodies (mAb). Vercellotti et al., 2019.

In light of the many questions remaining around the biological mechanisms underlying SCD and its various manifestations, there remains a need for methods of treatment that are beneficial for treating, inhibiting, reducing, or preventing SCD symptoms and manifestations in patients.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

Provided herein are methods of treating sickle cell disease, the method comprising administering to a mammalian subject in need thereof a therapeutically effective amount of a MASP-3 inhibitor, a MASP-2 inhibitor, or both a MASP-3 and a MASP-2 inhibitor. In some embodiments, the MASP-3 inhibitor inhibits alternative pathway complement activation in the subject and/or the MASP-2 inhibitor inhibits lectin pathway complement activation in the subject. In some embodiments, the MASP-3 and/or MASP-2 inhibitors are antibodies or antigen-binding fragments thereof.

Also provided herein are methods of treating, reducing, and/or preventing vaso-occlusion associated with sickle cell disease; of treating, reducing, and/or preventing inflammation associated with sickle cell disease; of treating, reducing, and/or preventing pain associated with sickle cell disease; and of treating, preventing, and/or reducing the number, duration, and/or severity of crisis episodes associated with sickle cell disease. In any of the foregoing methods, the method may comprise administering to a mammalian subject in need thereof a therapeutically effective amount of a MASP-3 inhibitor, a MASP-2 inhibitor, or both a MASP-3 and a MASP-2 inhibitor.

Further provided herein are compositions comprising a MASP-3 and/or MASP-2 inhibitor for use in treating, reducing, and/or preventing vaso-occlusion associated with sickle cell disease; for treating, reducing, and/or preventing inflammation associated with sickle cell disease; for treating, reducing and/or preventing pain associated with sickle cell diseases; and for treating, preventing, and/or reducing the number, duration, and/or severity of crisis episodes associated with sickle cell disease Any of the foregoing compositions may further comprise a pharmaceutically acceptable excipient.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

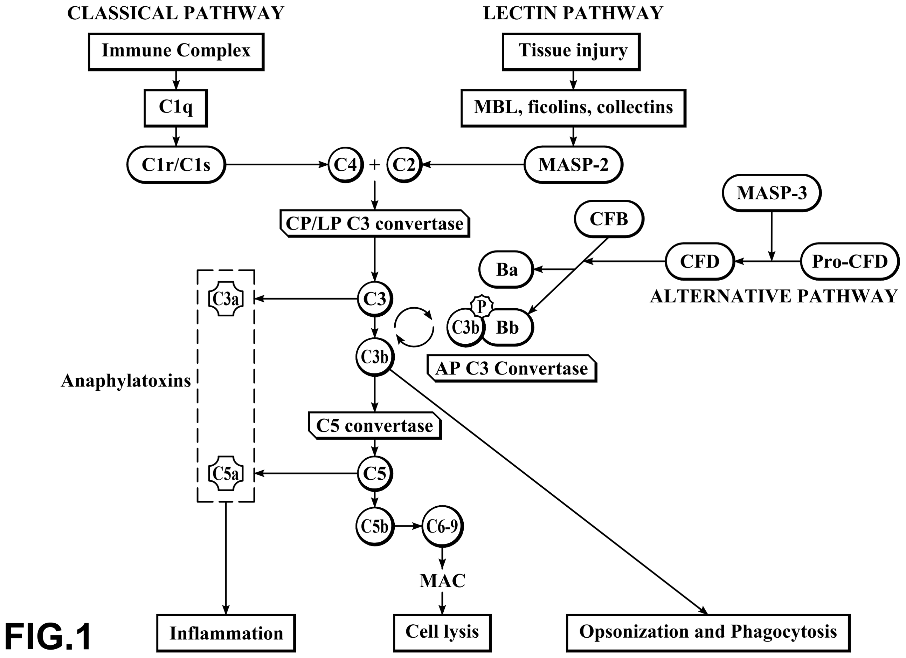

FIGURE 1 is a diagram illustrating the classical, lectin, and alternative complement pathways.

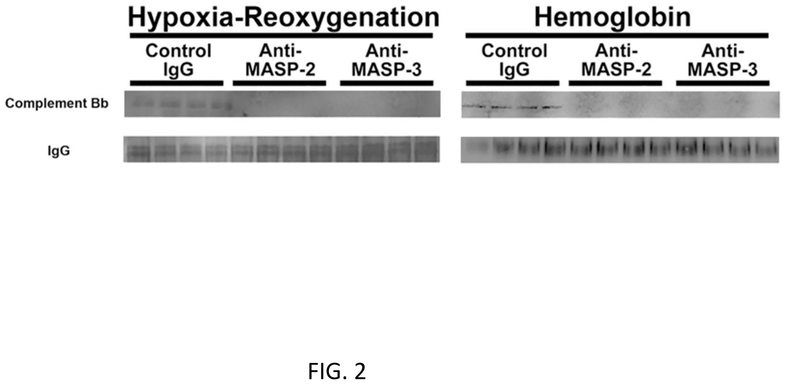

FIGURE 2 is an image of an immunoblot showing the detection (or lack thereof) of complement factor Bb in plasma of Townes-SS mice. The mice were infused with anti-MASP-3 antibody via the tail vein four days prior to challenge, or with anti-MASP-2 antibody or a control IgG antibody 30 minutes prior to challenge with hypoxia-reoxygenation (left panel) or hemoglobin infusion (right panel).

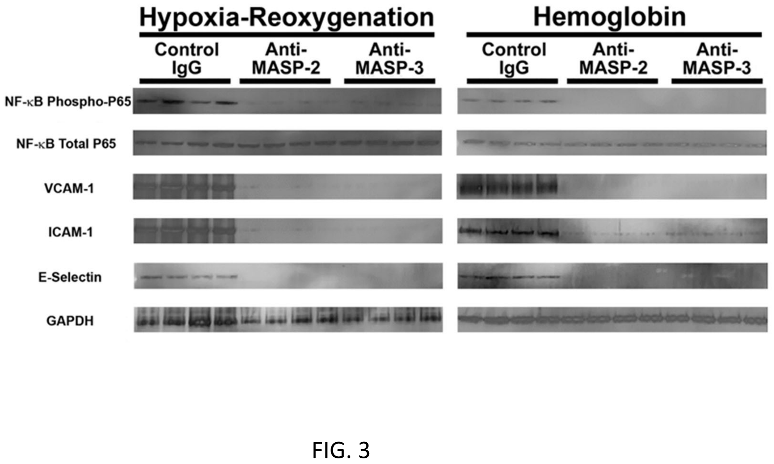

FIGURE 3 is an image of an immunoblot showing the detection (or lack thereof) of biomarkers related to inflammation in the livers of Townes-SS mice. The mice were infused with anti-MASP-3 antibody via the tail vein four days prior to challenge, or with anti-MASP-2 antibody or a control IgG antibody 30 minutes prior to challenge with hypoxia-reoxygenation (left panel) or hemoglobin infusion (right panel).

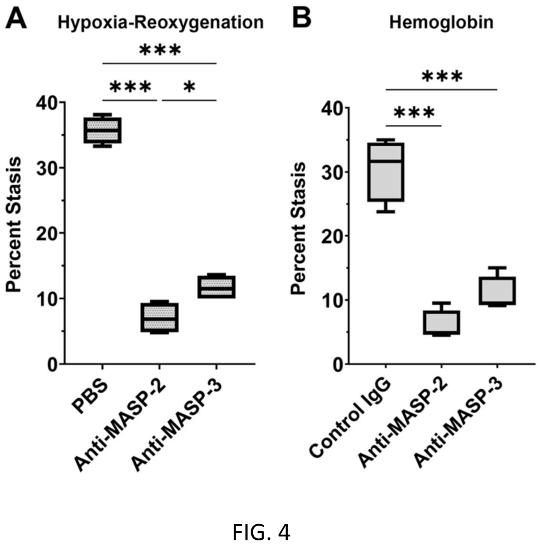

FIGURE 4 graphically illustrates the level of microvascular stasis in Townes-SS mice treated with anti-MASP-3 antibody four days prior to challenge or with anti-MASP-2 antibody 30 minutes prior to challenge with hypoxia-reoxygenation (left panel) or hemoglobin infusion (right panel). Controls using PBS alone (hypoxia-reoxygenation) or a control IgG antibody (hemoglobin) are also shown. ***P<0.001, one-way analysis of variance (ANOVA) (Dunnett's multiple comparisons test).

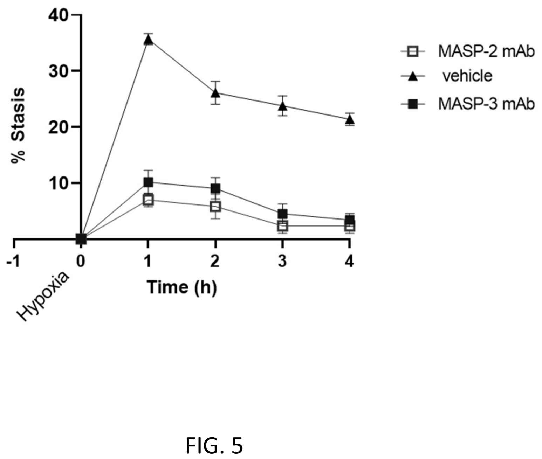

FIGURE 5 graphically illustrates the level of microvascular stasis in Townes-SS mice treated with anti-MASP3 antibody four days prior to challenge or with anti-MASP-2 antibody 30 minutes prior to challenge with hypoxia-reoxygenation over the course of four hours post-challenge. Control using PBS alone is also shown.

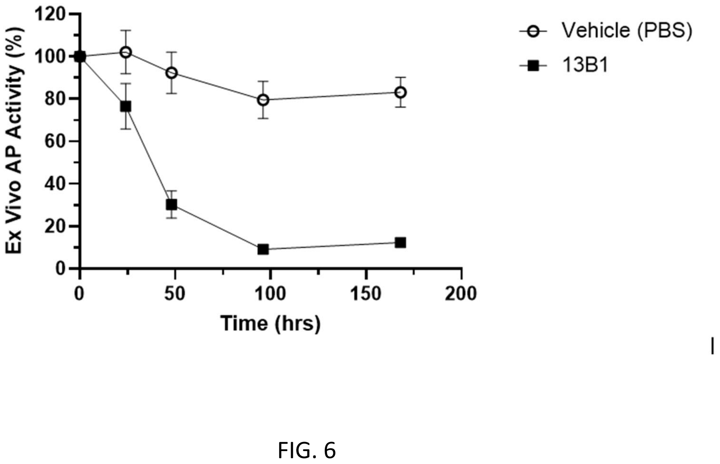

FIGURE 6 graphically illustrates the time course of inhibition of the alternative pathway (AP) of complement activation by MASP-3 antibody 13B1 in mice. 13B1 was administered by subcutaneous injection at a dose of 10 mg/kg of body weight. Serum was collected pre-dose and at the indicated time points after administration and was used to measure AP activity by cytometric detection of C3b/iC3b deposition on zymosan particles. Control using PBS alone is also shown.

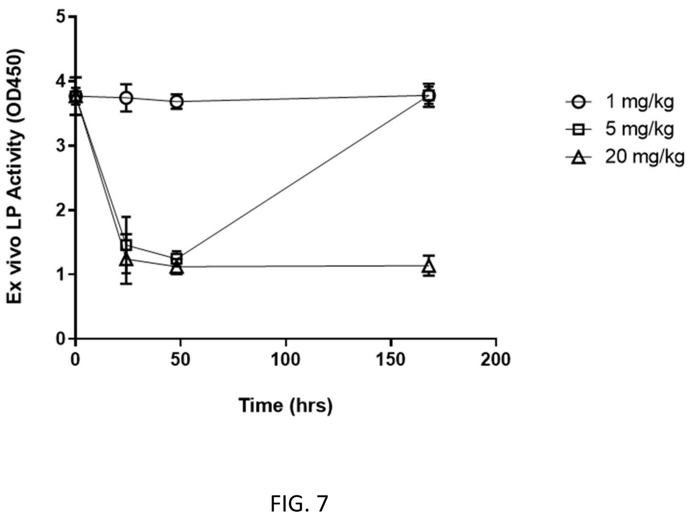

FIGURE 7 graphically illustrates the time course of inhibition of the lectin pathway (LP) of complement activation by MASP-2 antibody HG4 in mice. Mice were treated with HG4 administered by subcutaneous injection at a dose of 1 mg/kg, 5 mg/kg, or 20 mg/kg of body weight. Serum was collected pre-dose and at the indicated time points after administration and was used to measure LP activity by quantification of C3b deposition on mannan-coated ELISA plates.

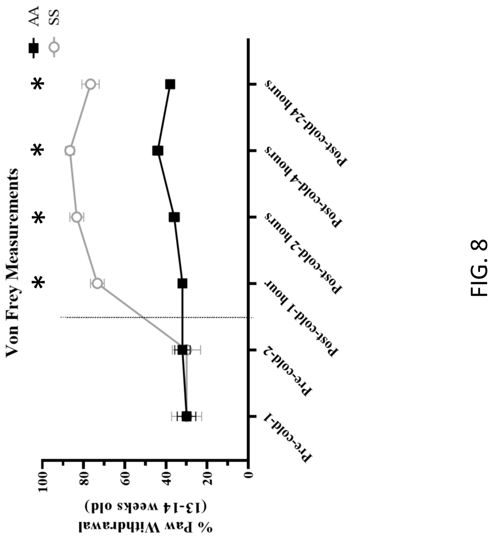

FIGURE 8 graphically illustrates the frequency of paw withdrawal by Townes-SS mice or Townes-AA mice (with wild-type human alpha and beta globin genes) before and after challenge with cold exposure. Mice were exposed to cold challenge of 10°C for two hours. Paw withdrawal was measured as percent paw withdrawal over ten repeated stimulations, five directed to each of the hind paws, with 0.8 gram Von Frey monofilament. Measurements were taken pre-challenge and at the indicated time points post-challenge. The vertical dashed line on the graph indicates cold challenge. The differences between wild type and SS mice at the time points indicated with asterisks are statistically significant.

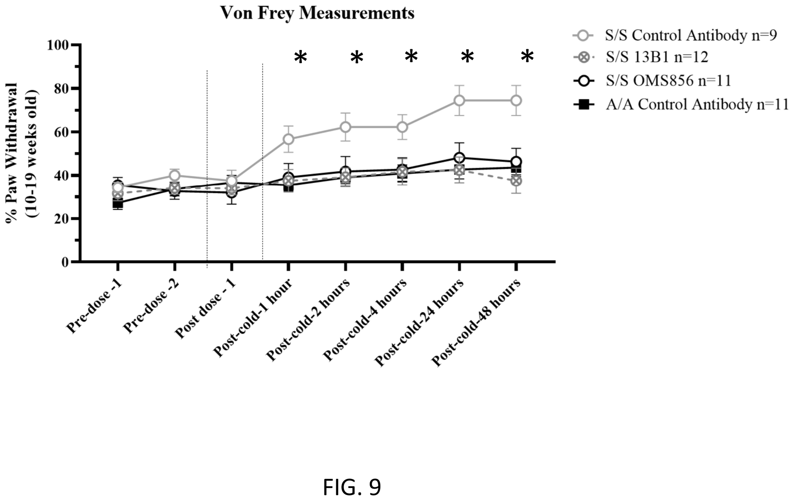

FIGURE 9 graphically illustrates the frequency of paw withdrawal by Townes-SS mice or Townes-AA mice before and after challenge with cold exposure where mice were administered anti-MASP-3 antibody 13B1, anti-MASP-2 antibody OMS856, or an isotype control antibody four days before cold challenge. Mice were exposed to cold challenge of 10°C for two hours. Antibody was administered by subcutaneous injection at a dose of 10 mg/kg of body weight. Measurements were taken pre-challenge and at the indicated time points post-challenge. The first vertical dashed line on the graph indicates dosing; the second vertical dashed line on the graph indicates cold challenge. The differences between mice administered either 13B1 or OMS856 and control antibody at the time points indicated with asterisks are statistically significant.

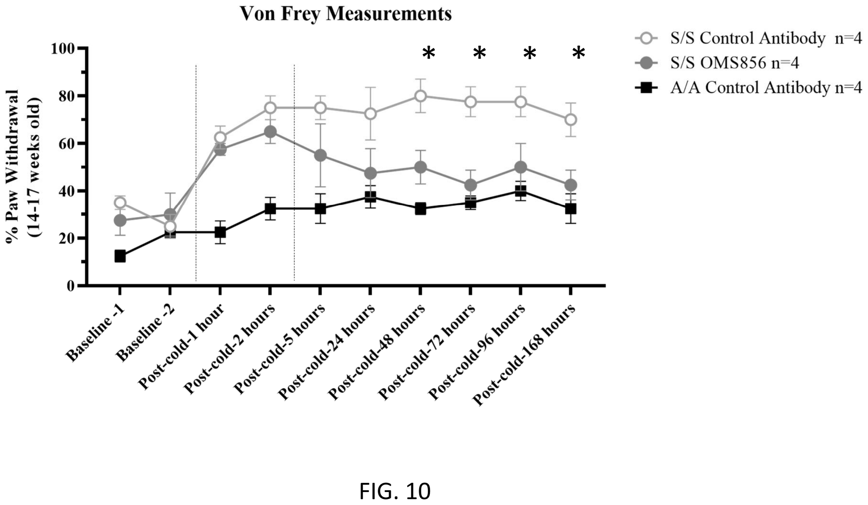

FIGURE 10 graphically illustrates the frequency of paw withdrawal by Townes-SS mice or Townes-AA mice before and after challenge with cold exposure where mice were administered anti-MASP-2 antibody OMS856 or an isotype control antibody four hours after cold challenge. Mice were exposed to cold challenge of 10°C for two hours. Antibody was administered by subcutaneous injection at a dose of 10 mg/kg of body weight. Measurements were taken pre-challenge and at the indicated time points post-challenge. The left vertical dashed line on the graph indicates cold challenge; the right vertical dashed line on the graph indicates dosing. The differences between mice administered OMS856 and control antibody at the time points indicated with asterisks are statistically significant.

### DETAILED DESCRIPTION

### I. Definitions

Unless specifically defined herein, all terms used herein have the same meaning as would be understood by those of ordinary skill in the art of the present invention. The following definitions are provided in order to provide clarity with respect to the terms as they are used in the specification and claims to describe the present invention. Additional definitions are set forth throughout this disclosure.

In the present descriptions, any concentration range, percentage range, ratio range, or integer range is to be understood to include the value of any integer within the recited range and, when appropriate, fractions thereof (such as one tenth and one hundredth of an integer), unless otherwise indicated or evident from the context. Any number range recited herein relating to any physical feature, such as polymer subunits, size, or thickness, is to be understood to include any integer within the recited range and, when appropriate, fractions thereof, unless otherwise indicated or evident from the context. As used herein, the term "about" is meant to specify that the range or value provided may vary by ±10% of the indicated range or value, unless otherwise indicated.

It should be understood that the terms "a", "an", and "the" as used herein refer to one or more of the referenced components. The use of the alternative (e.g., "or") should be understood to mean either one, both, or any combination of the alternatives. As used herein, the terms "include", "have", and "comprise" are used synonymously, which terms and variants thereof are intended to be construed as non-limiting.

"Optional" or "optionally" means that the subsequently described element, component, event, or circumstance may or may not occur, and that the description includes instances in which the element component, event, or circumstance occurs and instances in which it does not. It should be understood that the individual constructs or groups of constructs derived from the various combinations of the structures and subunits described herein are disclosed by the present application to the same extent as if each construct or group of constructs was set forth individually. Thus, selection of particular structures or particular subunits is within the scope of the present disclosure.

The term "consisting essentially of" is not equivalent to "comprising" and refers to the specified materials or steps of a claim, or to those that do not materially affect the basic characteristics of a claimed subject matter. For example, a protein domain, region, or module (e.g., a binding domain) or a protein "consists essentially of" a particular amino acid sequence when the amino acid sequence of a domain, region, module, or protein includes extensions, deletions, mutations, or a combination thereof (e.g., amino acids at the amino- or carboxy-terminus or between domains) that, in combination, contribute to at most 20% (e.g., at most 15%, 10%, 8%, 6%, 5%, 4%, 3%, 2% or 1%) of the length of a domain, region, module, or protein and do not substantially affect (i.e., do not reduce the activity by more than 50%, such as no more than 40%, 30%, 25%, 20%, 15%, 10%, 5%, or 1%) the activity of the domain(s), region(s), module(s), or protein (e.g., the target binding affinity of a binding protein).

As used herein, the terms "treat", "treatment", or "ameliorate" refer to medical management of a disease, disorder, or condition of a subject. In general, an appropriate dose or treatment regimen comprising a targeted complement-activating molecule or composition of the present disclosure is administered in an amount sufficient to elicit a therapeutic or prophylactic benefit. Therapeutic or prophylactic/preventive benefit includes improved clinical outcome; lessening or alleviation of symptoms or manifestations associated with a disease; decreased occurrence of symptoms or manifestations; improved quality of life; longer disease-free status; diminishment of extent of disease, stabilization of disease state; delay or prevention of disease progression; remission; survival; prolonged survival; or any combination thereof.

A "therapeutically effective amount" or "effective amount" of a targeted complement-activating molecule, polynucleotide, vector, host cell, or composition of this disclosure refers to an amount of the composition or molecule sufficient to result in a therapeutic effect, including improved clinical outcome; lessening or alleviation of symptoms or manifestations associated with a disease; decreased occurrence of symptoms or manifestations; improved quality of life; longer disease-free status; diminishment of extent of disease, stabilization of disease state; delay of disease progression; remission; survival; or prolonged survival in a statistically significant manner. When referring to an individual active ingredient, administered alone, a therapeutically effective amount refers to the effects of that ingredient or a cell expressing that ingredient alone. When referring to a combination, a therapeutically effective amount refers to the combined amounts of active ingredients or combined adjunctive active ingredient with a cell expressing an active ingredient that results in a therapeutic effect, whether administered serially, sequentially, or simultaneously.

As used herein, "a subject" includes all mammals, including without limitation humans, non-human primates, dogs, cats, horses, sheep, goats, cows, rabbits, pigs, and rodents. A subject may be male or female, and can be any suitable age, including infant, juvenile, adolescent, adult, and geriatric subjects.

As used herein, "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refer to compounds that have the same basic chemical structure as a naturally occurring amino acid, i.e., an α-carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refer to chemical compounds that have a structure that is different from the general chemical structure of an amino acid, but that functions in a manner similar to a naturally occurring amino acid.

As used herein, "mutation" refers to a change in the sequence of a nucleic acid molecule or polypeptide molecule as compared to a reference or wild-type nucleic acid molecule or polypeptide molecule, respectively. A mutation can result in several different types of change in sequence, including substitution, insertion or deletion of nucleotide(s) or amino acid(s).

In the broadest sense, the naturally occurring amino acids can be divided into groups based upon the chemical characteristic of the side chain of the respective amino acids. By "hydrophobic" amino acid is meant either Ile, Leu, Met, Phe, Trp, Tyr, Val, Ala, Cys or Pro. By "hydrophilic" amino acid is meant either Gly, Asn, Gln, Ser, Thr, Asp, Glu, Lys, Arg or His.

A "conservative substitution" refers to amino acid substitutions that do not significantly affect or alter binding characteristics of a particular protein. Generally, conservative substitutions are ones in which a substituted amino acid residue is replaced with an amino acid residue having a similar side chain. Conservative substitutions include a substitution found in one of the following groups: Group 1: Alanine (Ala or A), Glycine (Gly or G), Serine (Ser or S), Threonine (Thr or T); Group 2: Aspartic acid (Asp or D), Glutamic acid (Glu or Z); Group 3: Asparagine (Asn or N), Glutamine (Gln or Q); Group 4: Arginine (Arg or R), Lysine (Lys or K), Histidine (His or H); Group 5: Isoleucine (Ile or I), Leucine (Leu or L), Methionine (Met or M), Valine (Val or V); and Group 6: Phenylalanine (Phe or F), Tyrosine (Tyr or Y), Tryptophan (Trp or W). Additionally or alternatively, amino acids can be grouped into conservative substitution groups by similar function, chemical structure, or composition (e.g., acidic, basic, aliphatic, aromatic, or sulfur-containing). For example, an aliphatic grouping may include, for purposes of substitution, Gly, Ala, Val, Leu, and Ile. Other conservative substitutions groups include: sulfur-containing: Met and Cysteine (Cys or C); acidic: Asp, Glu, Asn, and Gln; small aliphatic, nonpolar or slightly polar residues: Ala, Ser, Thr, Pro, and Gly; polar, negatively charged residues and their amides: Asp, Asn, Glu, and Gln; polar, positively charged residues: His, Arg, and Lys; large aliphatic, nonpolar residues: Met, Leu, Ile, Val, and Cys; and large aromatic residues: Phe, Tyr, and Trp. Additional information can be found in Creighton (1984) Proteins, W.H. Freeman and Company.

As used herein, "protein" or "peptide" or "polypeptide" refers to a polymer of amino acid residues. Proteins apply to naturally occurring amino acid polymers, as well as to amino acid polymers in which one or more amino acid residue is an artificial chemical mimetic of a corresponding naturally occurring amino acid, and non-naturally occurring amino acid polymers. Variants of proteins, peptides, and polypeptides of this disclosure are also contemplated. In certain embodiments, variant proteins, peptides, and polypeptides comprise or consist of an amino acid sequence that is at least 70%, 75%, 80%, 85%, 90%, 91%, 92%, 93%, 94%, 95%, 96%, 97%, 98%, 99%, or 99.9% identical to an amino acid sequence of a defined or reference amino acid sequence as described herein.

"Nucleic acid molecule" or "oligonucleotide" or "polynucleotide" or "polynucleic acid" refers to an oligomeric or polymeric compound including covalently linked nucleotides, which can be made up of natural subunits (e.g., purine or pyrimidine bases) or non-natural subunits (e.g., morpholine ring). Purine bases include adenine, guanine, hypoxanthine, and xanthine, and pyrimidine bases include uracil, thymine, and cytosine. Nucleic acid molecules include polyribonucleic acid (RNA), which includes, for example, mRNA, microRNA, siRNA, viral genomic RNA, and synthetic RNA, and polydeoxyribonucleic acid (DNA), which includes, for example, cDNA, genomic DNA, and synthetic DNA. Both RNA and DNA may be single or double stranded. If single-stranded, the nucleic acid molecule may be the coding strand or non-coding (anti-sense) strand. A nucleic acid molecule encoding an amino acid sequence includes all nucleotide sequences that encode the same amino acid sequence. Some versions of the nucleotide sequences may also include intron(s) to the extent that the intron(s) would be removed through co- or post-transcriptional mechanisms. In other words, different nucleotide sequences may encode the same amino acid sequence as the result of the redundancy or degeneracy of the genetic code, or by splicing.

Variants of nucleic acid molecules of this disclosure are also contemplated. Variant nucleic acid molecules are at least 70%, 75%, 80%, 85%, 90%, and are preferably 95%, 96%, 97%, 98%, 99%, or 99.9% identical a nucleic acid molecule of a defined or reference polynucleotide as described herein, or that hybridize to a polynucleotide under stringent hybridization conditions of 0.015M sodium chloride, 0.0015M sodium citrate at about 65-68°C or 0.015M sodium chloride, 0.0015M sodium citrate, and 50% formamide at about 42°C. Nucleic acid molecule variants retain the capacity to encode a binding domain thereof having a functionality described herein, such as binding a target molecule.

"Percent sequence identity" refers to a relationship between two or more sequences, as determined by comparing the sequences. Preferred methods to determine sequence identity are designed to give the best match between the sequences being compared. For example, the sequences are aligned for optimal comparison purposes (e.g., gaps can be introduced in one or both of a first and a second amino acid or nucleic acid sequence for optimal alignment). Further, non-homologous sequences may be disregarded for comparison purposes. The percent sequence identity referenced herein is calculated over the length of the reference sequence, unless indicated otherwise. Methods to determine sequence identity and similarity can be found in publicly available computer programs. Sequence alignments and percent identity calculations may be performed using a BLAST program (e.g., BLAST 2.0, BLASTP, BLASTN, or BLASTX), or Megalign (DNASTAR) software. The mathematical algorithm used in the BLAST programs can be found in Altschul et al., Nucleic Acids Res. 25:3389-3402, 1997. Appropriate parameters for measuring alignment, including any algorithms needed to achieve maximal alignment over the full-length of the sequences being compared can be determined by known methods.

The term "isolated" means that the material is removed from its original environment (e.g., the natural environment if it is naturally occurring). For example, a naturally occurring nucleic acid or polypeptide present in a living animal is not isolated, but the same nucleic acid or polypeptide, separated from some or all of the co-existing materials in the natural system, is isolated. Such a nucleic acid could be part of a vector and/or such a nucleic acid or polypeptide could be part of a composition (e.g., a cell lysate), and still be isolated in that such vector or composition is not part of the natural environment for the nucleic acid or polypeptide. "Isolated" can, in some embodiments, also describe an antibody, antigen-binding fragment, polynucleotide, vector, host cell, or composition that is outside of a human body.

The term "gene" means the segment of DNA or RNA involved in producing a polypeptide chain; in certain contexts, it includes regions preceding and following the coding region (e.g., 5' untranslated region (UTR) and 3' UTR) as well as intervening sequences (introns) between individual coding segments (exons).

A "functional variant" refers to a polypeptide or polynucleotide that is structurally similar or substantially structurally similar to a parent or reference compound of this disclosure, but differs slightly in composition (e.g., one or more base, atom or functional group is different, added, or removed), such that the polypeptide or encoded polypeptide is capable of performing at least one function of the parent polypeptide with at least 50% efficiency, preferably at least 55%, 60%, 70%, 75%, 80%, 85%, 90%, 95%, 96%, 97%, 98%, 99%, 99.9%, 100% level of activity of the parent polypeptide, or a level of activity greater than that of the parent polypeptide. In other words, a functional variant of a polypeptide or encoded polypeptide of this disclosure has "similar binding," "similar affinity" or "similar activity" when the functional variant displays an improvement in performance, or no more than a 50% reduction in performance, in a selected assay as compared to the parent or reference polypeptide, such as an assay for measuring enzymatic activity or binding affinity.

As used herein, a "functional portion" or "functional fragment" refers to a polypeptide or polynucleotide that comprises only a domain, portion or fragment of a parent or reference compound, and the polypeptide or encoded polypeptide retains at least 50% activity associated with the domain, portion or fragment of the parent or reference compound, preferably at least 55%, 60%, 70%, 75%, 80%, 85%, 90%, 95%, 96%, 97%, 98%, 99%, 99.9%, 100% level of activity of the parent polypeptide, or a level of activity greater than that of the parent polypeptide, or provides a biological benefit (e.g., effector function). A "functional portion" or "functional fragment" of a polypeptide or encoded polypeptide of this disclosure has "similar binding" or "similar activity" when the functional portion or fragment displays an improvement in performance, or no more than a 50% reduction in performance, in a selected assay as compared to the parent or reference polypeptide (preferably no more than 20% or 10% reduction, or no more than a log difference as compared to the parent or reference with regard to affinity).

As used herein, the term "engineered," "recombinant," or "non-natural" refers to an organism, microorganism, cell, protein, polypeptide, nucleic acid molecule, or vector that includes at least one genetic alteration or has been modified by introduction of an exogenous or heterologous nucleic acid molecule, wherein such alterations or modifications are introduced by genetic engineering (i.e., human intervention). Genetic alterations include, for example, modifications introducing expressible nucleic acid molecules encoding functional RNA, proteins, fusion proteins or enzymes, or other nucleic acid molecule additions, deletions, substitutions, or other functional disruption of a cell's genetic material. Additional modifications include, for example, non-coding regulatory regions in which the modifications alter expression of a polynucleotide, gene, or operon.

As used herein, "heterologous" or "non-endogenous" or "exogenous" refers to any gene, protein, compound, nucleic acid molecule, or activity that is not native to a host cell or a subject, or any gene, protein, compound, nucleic acid molecule, or activity native to a host cell or a subject that has been altered. Heterologous, non-endogenous, or exogenous includes genes, proteins, compounds, or nucleic acid molecules that have been mutated or otherwise altered such that the structure, activity, or both is different as between the native and altered genes, proteins, compounds, or nucleic acid molecules. In certain embodiments, heterologous, non-endogenous, or exogenous genes, proteins, or nucleic acid molecules (e.g., receptors, ligands, etc.) may not be endogenous to a host cell or a subject, but instead nucleic acids encoding such genes, proteins, or nucleic acid molecules may have been added to a host cell by conjugation, transformation, transfection, electroporation, or the like, wherein the added nucleic acid molecule may integrate into a host cell genome or can exist as extra-chromosomal genetic material (e.g., as a plasmid or other self-replicating vector). The term "homologous" or "homolog" refers to a gene, protein, compound, nucleic acid molecule, or activity found in or derived from a host cell, species, or strain. For example, a heterologous or exogenous polynucleotide or gene encoding a polypeptide may be homologous to a native polynucleotide or gene and encode a homologous polypeptide or activity, but the polynucleotide or polypeptide may have an altered structure, sequence, expression level, or any combination thereof. A non-endogenous polynucleotide or gene, as well as the encoded polypeptide or activity, may be from the same species, a different species, or a combination thereof.

In certain embodiments, a nucleic acid molecule or portion thereof native to a host cell will be considered heterologous to the host cell if it has been altered or mutated, or a nucleic acid molecule native to a host cell may be considered heterologous if it has been altered with a heterologous expression control sequence or has been altered with an endogenous expression control sequence not normally associated with the nucleic acid molecule native to a host cell. In addition, the term "heterologous" can refer to a biological activity that is different, altered, or not endogenous to a host cell. As described herein, more than one heterologous nucleic acid molecule can be introduced into a host cell as separate nucleic acid molecules, as a plurality of individually controlled genes, as a polycistronic nucleic acid molecule, as a single nucleic acid molecule encoding an antibody or antigen-binding fragment (or other polypeptide), or any combination thereof.

As used herein, the term "endogenous" or "native" refers to a polynucleotide, gene, protein, compound, molecule, or activity that is normally present in a host cell or a subject.

The term "expression", as used herein, refers to the process by which a polypeptide is produced based on the encoding sequence of a nucleic acid molecule, such as a gene. The process may include transcription, post-transcriptional control, post-transcriptional modification, translation, post-translational control, posttranslational modification, or any combination thereof. An expressed nucleic acid molecule is typically operably linked to an expression control sequence (e.g., a promoter).

The term "operably linked" refers to the association of two or more nucleic acid molecules on a single nucleic acid fragment so that the function of one is affected by the other. For example, a promoter is operably linked with a coding sequence when it is capable of affecting the expression of that coding sequence (i.e., the coding sequence is under the transcriptional control of the promoter). "Unlinked" means that the associated genetic elements are not closely associated with one another and the function of one does not affect the other.

As described herein, more than one heterologous nucleic acid molecule can be introduced into a host cell as separate nucleic acid molecules, as a plurality of individually controlled genes, as a polycistronic nucleic acid molecule, as a single nucleic acid molecule encoding a protein (e.g., a heavy chain of an antibody), or any combination thereof. When two or more heterologous nucleic acid molecules are introduced into a host cell, it is understood that the two or more heterologous nucleic acid molecules can be introduced as a single nucleic acid molecule (e.g., on a single vector), on separate vectors, integrated into the host chromosome at a single site or multiple sites, or any combination thereof. The number of referenced heterologous nucleic acid molecules or protein activities refers to the number of different encoding nucleic acid molecules or the number of different protein activities, not the number of separate nucleic acid molecules introduced into a host cell.

The term "construct" refers to any polynucleotide that contains a recombinant nucleic acid molecule (or, when the context clearly indicates, a fusion protein of the present disclosure). A (polynucleotide) construct may be present in a vector (e.g., a bacterial vector, a viral vector) or may be integrated into a genome. A "vector" is a nucleic acid molecule that is capable of transporting another nucleic acid molecule. Vectors may be, for example, plasmids, cosmids, viruses, an RNA vector or a linear or circular DNA or RNA molecule that may include chromosomal, non-chromosomal, semi-synthetic or synthetic nucleic acid molecules. Vectors of the present disclosure also include transposon systems (e.g., Sleeping Beauty, see, e.g., Geurts et al., Mol. Ther. 8:108, 2003: Mátés et al., Nat. Genet. 41:753, 2009). Exemplary vectors are those capable of autonomous replication (episomal vector), capable of delivering a polynucleotide to a cell genome (e.g., viral vector), or capable of expressing nucleic acid molecules to which they are linked (expression vectors).

As used herein, "expression vector" or "vector" refers to a DNA construct containing a nucleic acid molecule that is operably linked to a suitable control sequence capable of effecting the expression of the nucleic acid molecule in a suitable host. Such control sequences typically include a promoter to effect transcription, an optional operator sequence to control such transcription, a sequence encoding suitable mRNA ribosome binding sites, and sequences which control termination of transcription and translation. The vector may be a plasmid, a phage particle, a virus, or simply a potential genomic insert. Once transformed into a suitable host, the vector may replicate and function independently of the host genome, or may, in some instances, integrate into the genome itself or deliver the polynucleotide contained in the vector into the genome without the vector sequence. In the present specification, "plasmid," "expression plasmid," "virus," and "vector" are often used interchangeably.

The term "introduced" in the context of inserting a nucleic acid molecule into a cell, means "transfection", "transformation," or "transduction" and includes reference to the incorporation of a nucleic acid molecule into a eukaryotic or prokaryotic cell wherein the nucleic acid molecule may be incorporated into the genome of a cell (e.g., chromosome, plasmid, plastid, or mitochondrial DNA), converted into an autonomous replicon, or transiently expressed (e.g., transfected mRNA).

In certain embodiments, polynucleotides of the present disclosure may be operatively linked to certain elements of a vector. For example, polynucleotide sequences that are needed to affect the expression and processing of coding sequences to which they are ligated may be operatively linked. Expression control sequences may include appropriate transcription initiation, termination, promoter, and enhancer sequences; efficient RNA processing signals such as splicing and polyadenylation signals; sequences that stabilize cytoplasmic mRNA; sequences that enhance translation efficiency (i.e., Kozak consensus sequences); sequences that enhance protein stability; and possibly sequences that enhance protein secretion. Expression control sequences may be operatively linked if they are contiguous with the gene of interest and expression control sequences that act in trans or at a distance to control the gene of interest may also be considered operatively linked.

In certain embodiments, the vector comprises a plasmid vector or a viral vector (e.g., a lentiviral vector or a γ-retroviral vector). Viral vectors include retrovirus, adenovirus, parvovirus (e.g., adeno-associated viruses), coronavirus, negative strand RNA viruses such as ortho-myxovirus (e.g., influenza virus), rhabdovirus (e.g., rabies and vesicular stomatitis virus), paramyxovirus (e.g., measles and Sendai), positive strand RNA viruses such as picornavirus and alphavirus, and double-stranded DNA viruses including adenovirus, herpesvirus (e.g., Herpes Simplex virus types 1 and 2, Epstein-Barr virus, cytomegalovirus), and poxvirus (e.g., vaccinia, fowlpox, and canarypox). Other viruses include, for example, Norwalk virus, togavirus, flavivirus, reoviruses, papovavirus, hepadnavirus, and hepatitis virus. Examples of retroviruses include avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields et al., Eds., Lippincott-Raven Publishers, Philadelphia, 1996). Methods of using retroviral and lentiviral viral vectors and packaging cells for transducing mammalian host cells with viral particles containing transgenes are known in the art and have been previous described, for example, in: U.S. Patent 8,119,772; Walchli et al., PLoS One 6:327930, 2011; Zhao et al., J. Immunol. 174:4415, 2005; Engels et al., Hum. Gene Ther. 14:1155, 2003; Frecha et al., Mol. Ther. 18:1748, 2010; and Verhoeyen et al., Methods Mol. Biol. 506:97, 2009. Retroviral and lentiviral vector constructs and expression systems are also commercially available. Other viral vectors also can be used for polynucleotide delivery including DNA viral vectors, including, for example adenovirus-based vectors and adeno-associated virus (AAV)-based vectors; vectors derived from herpes simplex viruses (HSVs), including amplicon vectors, replication-defective HSV and attenuated HSV (Krisky et al., Gene Ther. 5:1517, 1998).

Other vectors that can be used with the compositions and methods of this disclosure include those derived from baculoviruses and α-viruses. (Jolly, D J. 1999. Emerging Viral Vectors. pp 209-40 in Friedmann T. ed. The Development of Human Gene Therapy. New York: Cold Spring Harbor Lab), or plasmid vectors (such as Sleeping Beauty or other transposon vectors).

When a viral vector genome comprises a plurality of polynucleotides to be expressed in a host cell as separate transcripts, the viral vector may also comprise additional sequences between the two (or more) transcripts allowing for bicistronic or multicistronic expression. Examples of such sequences used in viral vectors include internal ribosome entry sites (IRES), furin cleavage sites, viral 2A peptide, or any combination thereof.

Plasmid vectors, including DNA-based plasmid vectors for expression of one or more proteins in vitro or for direct administration to a subject, are also known in the art. Such vectors may comprise a bacterial origin of replication, a viral origin of replication, genes encoding components required for plasmid replication, and/or one or more selection markers, and may also contain additional sequences allowing for bicistronic or multicistronic expression.

As used herein, the term "host" refers to a cell or microorganism targeted for genetic modification with a heterologous nucleic acid molecule to produce a polypeptide of interest (e.g., an antibody of the present disclosure).

A host cell may include any individual cell or cell culture which may receive a vector or the incorporation of nucleic acids or express proteins. The term also encompasses progeny of the host cell, whether genetically or phenotypically the same or different. Suitable host cells may depend on the vector and may include mammalian cells, animal cells, human cells, simian cells, insect cells, yeast cells, and bacterial cells. These cells may be induced to incorporate the vector or other material by use of a viral vector, transformation via calcium phosphate precipitation, DEAE-dextran, electroporation, microinjection, or other methods. See, for example, Sambrook et al., Molecular Cloning: A Laboratory Manual 2d ed. (Cold Spring Harbor Laboratory, 1989).

"Antigen", as used herein, refers to an immunogenic molecule that provokes an immune response. This immune response may involve antibody production, activation of specific immunologically competent cells, activation of complement, antibody dependent cytotoxicity, or any combination thereof. An antigen (immunogenic molecule) may be, for example, a peptide, glycopeptide, polypeptide, glycopolypeptide, polynucleotide, polysaccharide, lipid, or the like. It is readily apparent that an antigen can be synthesized, produced recombinantly, or derived from a biological sample. Exemplary biological samples that can contain one or more antigens include tissue samples, stool samples, cells, biological fluids, or combinations thereof. Antigens can be produced by cells that have been modified or genetically engineered to express an antigen. Antigens can also be present in or on an infectious agent, such as present in a virion, or expressed or presented on the surface of a cell infected by infectious agent.

The term "epitope" or "antigenic epitope" includes any molecule, structure, amino acid sequence, or protein determinant that is recognized and specifically bound by a cognate binding molecule, such as an immunoglobulin, or other binding molecule, domain, or protein. Epitopic determinants generally contain chemically active surface groupings of molecules, such as amino acids or sugar side chains, and can have specific three-dimensional structural characteristics, as well as specific charge characteristics. Where an antigen is or comprises a peptide or protein, the epitope can be comprised of consecutive amino acids (e.g., a linear epitope), or can be comprised of amino acids from different parts or regions of the protein that are brought into proximity by protein folding (e.g., a discontinuous or conformational epitope), or non-contiguous amino acids that are in close proximity irrespective of protein folding.

The term "antibody" refers to an immunoglobulin molecule consisting of one or more polypeptides that specifically binds an antigen through at least one epitope recognition site. For example, the term "antibody" encompasses an intact antibody comprising at least two heavy chains and two light chains connected by disulfide bonds, as well as any antigen-binding portion or fragment of an intact antibody that has or retains the ability to bind to the antigen target molecule recognized by the intact antibody, such as an scFv, Fab, or Fab'2 fragment. The term also encompasses full-length or fragments of antibodies of any class or sub-class, including IgG and sub-classes thereof (such as IgG1, IgG2, IgG3, and IgG4), IgM, IgE, IgA, and IgD.

The term "antibody" is used herein in the broadest sense, encompassing antibodies and antibody fragments thereof, derived from any antibody producing mammal (e.g., mouse, rat, rabbit, and primate including human), or from a hybridoma, phage selection, recombinant expression or transgenic animals (or other methods of producing antibodies or antibody fragments). It is not intended that the term "antibody" be limited as regards to the source of the antibody or manner in which it is made (e.g., by hybridoma, phage selection, recombinant expression, transgenic animal, peptide synthesis, etc.). Exemplary antibodies include polyclonal, monoclonal and recombinant antibodies; multispecific antibodies (e.g., bispecific antibodies); humanized antibodies; fully human antibodies, murine antibodies; chimeric, mouse human, mouse primate, primate human monoclonal antibodies; and anti-idiotype antibodies, and may be any intact molecule or fragment thereof. As used herein, the term "antibody" encompasses not only intact polyclonal or monoclonal antibodies, but also fragments thereof (such as dAb, Fab, Fab', F(ab')2, Fv), single chain (ScFv), synthetic variants thereof, naturally occurring variants, fusion proteins comprising an antibody portion with an antigen-binding fragment of the required specificity, humanized antibodies, chimeric antibodies, and any other modified configuration of the immunoglobulin molecule that comprises an antigen-binding site or fragment (epitope recognition site) of the required specificity. The term encompasses genetically engineered and- or otherwise modified forms of immunoglobulins such as intrabodies, peptibodies, diabodies, triabodies, tetrabodies, tandem di-scFv, tandem tri-scFv, and the like, including antigen-binding fragments thereof.

The terms "VH" and "VL" refer to the variable binding regions from an antibody heavy chain and an antibody light chain, respectively. A VL may be a kappa class chain or a lambda class chain. The variable binding regions comprise discrete, well-defined sub-regions known as complementarity determining regions (CDRs) and framework regions (FRs). The CDRs are located within a hypervariable region (HVR) of the antibody and refer to sequences of amino acids within antibody variable regions which, in general, together confer the antigen specificity and/or binding affinity of the antibody. Consecutive CDRs (i.e., CDR1 and CDR2, and CDR2 and CDR3) are separated from one another in primary structure by a framework region.

As used herein, a "chimeric antibody" is a recombinant protein that contains the variable domains and complementarity determining regions derived from a non-human species (e.g., rodent) antibody, while the remainder of the antibody molecule is derived from a human antibody. In some embodiments, a chimeric antibody is comprised of an antigen-binding fragment of one antibody operably linked or otherwise fused to a heterologous Fc portion of a different antibody. For example, a mouse-human chimeric antibody may comprise an antigen-binding fragment of a mouse antibody fused to an Fc portion derived from a human antibody. In some embodiments, the heterologous Fc domain may be from a different Ig class from the parent antibody, including IgA (including subclasses IgA1 and IgA2), IgD, IgE, IgG (including subclasses IgG1, IgG2, IgG3 and IgG4) and IgM.

As used herein, a "humanized antibody" is a molecule, generally prepared using recombinant techniques, having an antigen-binding site derived from an immunoglobulin from a non-human species and the remaining immunoglobulin structure of the molecule based upon the structure and/or sequence of a human immunoglobulin. A humanized antibody differs from a chimeric antibody in that typically only the CDRs from the non-human species are used, grafted onto appropriate framework regions in a human variable domain. Antigen binding sites may be wild type or may be modified by one or more amino acid substitutions. In some embodiments, humanized antibodies preserve all CDR sequences (for example, a humanized mouse antibody which contains all six CDRs from the mouse antibodies). In other embodiments, humanized antibodies have one or more CDRs (one, two, three, four, five, six) which are altered with respect to the original antibody, which are also termed one or more CDRs "derived from" one or more CDRs from the original antibody.

As used herein, the term "antibody fragment" refers to a portion derived from or related to a full-length antibody, generally including the antigen binding or variable region thereof. Illustrative examples of antibody fragments include Fab, Fab', F(ab)2, F(ab')2 and Fv fragments, scFv fragments, diabodies, linear antibodies, single chain antibody molecules and multispecific antibodies formed from antibody fragments.

As used herein, the term "antigen-binding fragment" refers to a polypeptide fragment that contains at least one CDR of an immunoglobulin heavy and/or light chains that specifically binds to the antigen to which the antibody was raised. An antigen-binding fragment may comprise 1, 2, 3, 4, 5, or all 6 CDRs of a VH and VL sequence from an antibody.

A "Fab" (fragment antigen binding) is the part of an antibody that binds to antigens and includes the variable region and CH1 of the heavy chain linked to the light chain via an inter-chain disulfide bond. Each Fab fragment is monovalent with respect to antigen binding, i.e., it has a single antigen-binding site. Pepsin treatment of an antibody yields a single large F(ab')2 fragment that roughly corresponds to two disulfide-linked Fab fragments having divalent antigen-binding activity and is still capable of cross-linking antigen. Both the Fab and F(ab')2 are examples of "antigen-binding fragments." Fab' fragments differ from Fab fragments by having a few additional residues at the carboxy terminus of the CH1 domain including one or more cysteines from the antibody hinge region. Fab'-SH is the designation herein for Fab' in which the cysteine residue(s) of the constant domains bear a free thiol group. F(ab')2 antibody fragments are often produced as pairs of Fab' fragments that have hinge cysteines between them. Other chemical couplings of antibody fragments are also known.

Fab fragments may be joined, e.g., by a peptide linker, to form a single chain Fab, also referred to herein as "scFab." In these embodiments, an inter-chain disulfide bond that is present in a native Fab may not be present, and the linker serves in full or in part to link or connect the Fab fragments in a single polypeptide chain. A heavy-chain derived Fab fragment (e.g., comprising, consisting of, or consisting essentially of VH + CH1, or "Fd") and a light chain-derived Fab fragment (e.g., comprising, consisting of, or consisting essentially of VL + CL) may be linked in any arrangement to form a scFab. For example, a scFab may be arranged, in N-terminal to C-terminal direction, according to (heavy chain Fab fragment - linker - light chain Fab fragment) or (light chain Fab fragment - linker - heavy chain Fab fragment).

"Fv" is a small antibody fragment that contains a complete antigen-recognition and antigen-binding site. This fragment generally consists of a dimer of one heavy- and one light-chain variable region domain in tight, non-covalent association. However, even a single variable domain (or half of an Fv comprising only three CDRs specific for an antigen) has the ability to recognize and bind antigen, although typically at a lower affinity than the entire binding site.

"Single-chain Fv" also abbreviated as "sFv" or "scFv", are antibody fragments that comprise the VH and VL antibody domains connected into a single polypeptide chain. The scFv polypeptide may comprise a polypeptide linker disposed between and linking the VH and VL domains that enables the scFv to retain or form the desired structure for antigen binding, although a linker is not always required. Such a peptide linker can be incorporated into a fusion polypeptide using standard techniques well known in the art. Additionally, or alternatively, Fv can have a disulfide bond formed between and stabilizing the VH and the VL. For a review of scFv, see Pluckthun in The Pharmacology of Monoclonal Antibodies, vol. 113, Rosenburg and Moore eds., Springer-Verlag, New York, pp. 269-315 (1994). In certain embodiments, the antibody or antigen-binding fragment comprises a scFv comprising a VH domain, a VL domain, and a peptide linker linking the VH domain to the VL domain. In particular embodiments, a scFv comprises a VH domain linked to a VL domain by a peptide linker, which can be in a VH-linker-VL orientation or in a VL-linker-VH orientation. Any scFv of the present disclosure may be engineered so that the C-terminal end of the VL domain is linked by a short peptide sequence to the N-terminal end of the VH domain, or vice versa (i.e., (N)VL(C)-linker-(N)VH(C) or (N)VH(C)-linker-(N)VL(C)). Alternatively, in some embodiments, a linker may be linked to an N-terminal portion or end of the VH domain, the VL domain, or both.

Peptide linker sequences for use in scFv or in other fusion proteins, such as the targeted complement-activating molecules described herein, may be chosen, for example, based on: (1) their ability to adopt a flexible extended conformation; (2) their inability or lack of ability to adopt a secondary structure that could interact with functional epitopes on the first and second polypeptides and/or on a target molecule; and/or (3) the lack or relative lack of hydrophobic or charged residues that might react with the polypeptides and/or target molecule. Other considerations regarding linker design (e.g., length) can include the conformation or range of conformations in which the VH and VL can form a functional antigen-binding site. In certain embodiments, peptide linker sequences contain, for example, Gly, Asn and Ser residues. Other near neutral amino acids, such as Thr and Ala, may also be included in a linker sequence. Other amino acid sequences which may be usefully employed as linker include those disclosed in Maratea et al., Gene 40:39 46(1985); Murphy et al., Proc. Natl. Acad. Sci. USA 83:8258 8262 (1986); U.S. Pat. No.4,935,233, and U.S. Pat. No. 4,751,180. Any suitable linker may be used, and in general can be about 3, 4, 5, 6, 7, 8, 9, 10, 11, 12, 13, 14, 15, 16, 17, 18, 19, 20, 21, 22, 15 23, 24, 25, 26, 27, 28, 29, 30, 40, 50, 60, 70, 80, 90, 100 amino acids in length, or less than about 200 amino acids in length, and will preferably comprise a flexible structure (can provide flexibility and room for conformational movement between two regions, domains, motifs, fragments, or modules connected by the linker), and will preferably be biologically inert and/or have a low risk of immunogenicity in a human.

Antibodies may be monospecific (e.g., binding to a single epitope) or multispecific (e.g., binding to multiple epitopes and/or target molecules). A bispecific or multispecific antibody or antigen-binding fragment may, in some embodiments, comprise one, two, or more antigen-binding domains (e.g., a VH and a VL). Two or more binding domains may be present that bind to the same or different epitopes, and a bispecific or multispecific antibody or antigen-binding fragment as provided herein can, in some embodiments, two or more binding domains, that bind to different antigens or pathogens altogether.

Antibodies and antigen-binding fragments may be constructed in various formats. Exemplary antibody formats disclosed in Spiess et al., Mol. Immunol. 67(2):95 (2015), and in Brinkmann and Kontermann, mAbs 9(2):182-212 (2017), which formats and methods of making the same are incorporated herein by reference and include, for example, Bispecific T cell Engagers (BiTEs), DARTs, Knobs-Into-Holes (KIH) assemblies, scFv-CH3-KIH assemblies, KIH Common Light-Chain antibodies, TandAbs, Triple Bodies, TriBi Minibodies, Fab-scFv, scFv-CH-CL-scFv, F(ab')2-scFv2, tetravalent HCabs, Intrabodies, CrossMabs, Dual Action Fabs (DAFs) (two-in-one or four-in-one), DutaMabs, DT-IgG, Charge Pairs, Fab-arm Exchange, SEEDbodies, Triomabs, LUZ-Y assemblies, Fcabs, κλ-bodies, orthogonal Fabs, DVD-Igs (e.g., US Patent No. 8,258,268, which formats are incorporated herein by reference in their entirety), IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig, Zybody, and DVI-IgG (four-in-one), as well as so-called FIT-Ig (e.g., PCT 5 Publication No. WO 2015/103072, which formats are incorporated herein by reference in their entirety), so-called WuxiBody formats (e.g., PCT Publication No. WO 2019/057122, which formats are incorporated herein by reference in their entirety), and so-called In-Elbow-Insert Ig formats (IEI-Ig; e.g., PCT Publication Nos. WO 2019/024979 and WO 2019/025391, which formats are incorporated herein by reference in their entirety).

An antibody or antigen-binding fragment may comprise two or more VH domains, two or more VL domains, or both (i.e., two or more VH domains and two or more VL domains). In particular embodiments, an antigen-binding fragment comprises the format (N-terminal to C-terminal direction) VH-linker-VL-linker-VH-linker-VL, wherein the two VH sequences can be the same or different and the two VL sequences can be the same or different. Such linked scFvs can include any combination of VH and VL domains arranged to bind to a given target, and in formats comprising two or more VH and/or two or more VL, one, two, or more different epitopes or antigens may be bound. It will be appreciated that formats incorporating multiple antigen-binding domains may include VH and/or VL sequences in any combination or orientation. For example, the antigen-binding fragment can comprise the format VL-linker-VH-linker-VL-linker-VH, VH-linker-VL-linker-VL-linker-VH, or VL-linker-VH-linker-VH-linker-VL.

As used herein, the modifier "monoclonal" indicates the character of the antibody as being obtained from a substantially homogenous population of antibodies, and is not intended to be limited as regards the source of the antibody or the manner in which it is made (e.g., by hybridoma, phage selection, recombinant expression, transgenic animals, etc.). The term "monoclonal antibody" encompasses not only intact monoclonal antibodies and full-length monoclonal antibodies, but also fragments thereof (such as Fab, Fab', F(ab')2, Fv), single chain (ScFv), variants thereof, fusion proteins comprising an antigen-binding portion, humanized monoclonal antibodies, chimeric monoclonal antibodies, and any other modified configuration of the immunoglobulin molecule that comprises an antigen-binding fragment (epitope recognition site) of the required specificity and the ability to bind to an epitope. Monoclonal antibodies can be obtained using any technique that provides for the production of antibody molecules by continuous cell lines in culture, such as the hybridoma method described by Kohler, G., et al., Nature 256:495, 1975, or they may be made by recombinant DNA methods (see, e.g., U.S. Patent No. 4,816,567 to Cabilly). Monoclonal antibodies may also be isolated from phage antibody libraries using the techniques described in Clackson, T., et al., Nature 352:624 628, 1991, and Marks, J.D., et al., J. Mol. Biol. 222:581 597, 1991. Such antibodies can be of any immunoglobulin class including IgG, IgM, IgE, IgA, IgD and any subclass thereof.

The recognized immunoglobulin polypeptides include the kappa and lambda light chains and the alpha, gamma (IgG1, IgG2, IgG3, IgG4), delta, epsilon and mu heavy chains, or equivalents in other species. Full-length immunoglobulin "light chains" (of about 25 kDa or about 214 amino acids) comprise a variable region of about 110 amino acids at the NH2 terminus and a kappa or lambda constant region at the COOH-terminus. Full-length immunoglobulin "heavy chains" (of about 50 kDa or about 446 amino acids) similarly comprise a variable region (of about 116 amino acids) and one of the aforementioned heavy chain constant regions, e.g., gamma (of about 330 amino acids).

The basic four-chain antibody unit is a heterotetrameric glycoprotein composed of two identical light (L) chains and two identical heavy (H) chains. An IgM antibody differs from this plan in that it consists of five of the basic heterotetramer units along with an additional polypeptide called the J chain, and therefore contains 10 antigen binding sites. Secreted IgA antibodies also differ from the basic structure in that they can polymerize to form polyvalent assemblages comprising two to five of the basic four-chain units along with a J chain. Each L chain is linked to an H chain by one covalent disulfide bond, while the two H chains are linked to each other by one or more by one or more disulfide bonds, depending on the H chain isotype. Each H and L chain also has regularly spaced intrachain disulfide bridges. The pairing of a VH and VL together forms a single antigen-binding site.

Each H chain has, at the N-terminus, a variable domain (VH) followed by three constant domains (CH1, CH2, CH3), in the case of alpha, gamma, and delta chains, or four CH domains (CH1, CH2, CH3, CH4), in the case of mu and epsilon chains.

Each L chain has, at the N-terminus, a variable domain (VL) followed by a constant domain (CL) at its other end. When an L chain and an H chain are paired, the VL is aligned with the VH and the CL is aligned with the first constant domain of the heavy chain (CH1). The L chain from any vertebrate species can be assigned to one of two types, called kappa (κ) and lambda (λ), based on the amino acid sequences of their constant domains (CL).

Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated alpha (α), delta (δ), epsilon (ε), gamma (γ) and mu (µ), respectively. The γ and α classes are further divided into subclasses on the basis of minor differences in CH sequence and function, for example, humans express the following subclasses: IgG1, IgG2, IgG3, IgG4, IgA1 and IgA2.

For the structure and properties of the different classes of antibodies, see, e.g., Basic and Clinical Immunology, 8th Edition, Daniel P. Stites, Abba I. Terr and Tristram G. Parslow (eds); Appleton and Lange, Norwalk, Conn., 1994, page 71 and Chapter 6.

The term "variable" refers to that fact that certain segments of the V domains differ extensively in sequence among antibodies. The V domain mediates antigen binding and defines specificity of a particular antibody for its particular antigen. However, the variability is not evenly distributed across the 110 amino acid span of the variable domains. Rather, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a beta-sheet configuration, connected by three hypervariable regions, which form loops connecting, and in some cases forming part of, the n-sheet structure. The hypervariable regions in each chain are held together in close proximity by the FRs and, with the hypervariable regions from the other chain, contribute to the formation of the antigen-binding site of antibodies (see Kabat, et al., Sequences of Proteins of Immunological Interest, 5th Ed. Public Health Service, National Institutes of Health, Bethesda, Md (1991)). The constant domains are not involved directly in binding an antibody to an antigen, but exhibit various effector functions.

As used herein, "effector functions" refer to those biological activities attributable to the Fc region of an antibody. Examples of antibody effector functions include participation in antibody-dependent cellular cytotoxicity (ADCC), Clq binding and complement-dependent cytotoxicity, Fc receptor binding, phagocytosis, down-regulation of cell surface receptors, and B cell activation. Modifications such as amino acid substitutions may be made to an Fc domain in order to modify (e.g., enhance or reduce) one or more functions of an Fc-containing polypeptide. Such functions include, for example, Fc receptor binding, antibody half-life modulation, ADCC function, protein A binding, protein G binding, and complement binding. Amino acid modifications that modify Fc functions include, for example, T250Q/M428L, M252Y/S254T/T256E, H433K/N434F, M428L/N434S, E233P/L234V/L235A/G236Δ/A327G/A330S/P331S, E333A, S239D/A330L/I332E, P257I/Q311, K326W/E333S, S239D/I332E/G236A, N297Q, K322A, S228P, L235E/E318A/K320A/K322A, L234A/L235A, and L234A/L235A/P329G mutations. Other Fc modifications and their effect on Fc function are known in the art.

As used herein, the term "hypervariable region" refers to the amino acid residues of an antibody that are responsible for antigen binding. The hypervariable region contains several "complementarity determining regions" (CDRs). The heavy chain comprises three CDR sequences (CDRH1, CDRH2, and CDRH3) and the light chain comprises three CDR sequences (CDRL1, CDRL2, and CDRL3). A variety of systems exist for identifying and numbering the amino acids that make up the CDRs. For example, the hypervariable region generally comprises CDRs at around about residues 24-34 (L1), 50-56 (L2) and 89-97 (L3) in the light chain variable domain, and at around about 31-35 (H1), 50-65 (H2) and 95-102 (H3) in the heavy chain variable domain when numbering in accordance with the Kabat numbering system as described in Kabat, et al., Sequences of Proteins of Immunological Interest, 5th Ed. Public Health Service, National Institutes of Health, Bethesda, Md (1991); and/or at about residues 24-34 (L1), 50-56 (L2) and 89-97 (L3) in the light chain variable domain, and 26-32 (H1), 52-56 (H2) and 95-102 (H3) in the heavy chain variable domain when numbered in accordance with the Chothia numbering system, as described in Chothia and Lesk, J. Mol. Biol. 196:901-917 (1987); and/or at about residues 27-38 (L1), 56-65 (L2) and 105-117 (L3) in the VL, and 27-38 (H1), 56-65 (H2), and 105-117 (H3) in the VH when numbered in accordance with the IMGT numbering system as described in Lefranc, J.P., et al., Nucleic Acids Res 27:209-212; Ruiz, M., et al., Nucleic Acids Res 28:219-221 (2000). Equivalent residue positions can be annotated and compared for different molecules using Antigen receptor Numbering and Receptor Classification (ANARCI) software tool (2016, Bioinformatics 15:298-300). Accordingly, identification of CDRs of an exemplary variable domain (VH or VL) sequence as provided herein according to one numbering scheme is not exclusive of an antibody comprising CDRs of the same variable domain as determined using a different numbering scheme.

As used herein, "specifically binds" refers to an antibody or antigen-binding fragment that binds to an antigen with a particular affinity, while not significantly associating or uniting with any other molecules or components in a sample. Affinity may be defined as an equilibrium association constant (Ka), calculated as the ratio of kon/koff, with units of 1/M or as an equilibrium dissociation constant Kd), calculated as the ratio of koff/kon with units of M.

In some contexts, antibody and antigen-binding fragments may be described with reference to affinity and/or to avidity for antigen. Unless otherwise indicated, avidity refers to the total binding strength of an antibody or antigen-binding fragment thereof to antigen, and reflects binding affinity, valency of the antibody or antigen-binding fragment (e.g., whether the antibody or antigen-binding fragment comprises one, two, three, four, five, six, seven, eight, nine, ten, or more binding sites), and, for example, whether another agent is present that can affect the binding (e.g., a non-competitive inhibitor of the antibody or antigen-binding fragment).

Each embodiment in this specification is to be applied mutatis mutandis to every other embodiment unless expressly stated otherwise. It is contemplated that any embodiment discussed in this specification can be implemented with respect to any method, kit, reagent, or composition of the invention, and vice versa. Furthermore, compositions of the invention can be used to achieve methods of the invention.

### II. Overview

Provided herein are compositions and methods for treating, inhibiting, reducing, or preventing sickle cell disease and/or the various manifestations of sickle cell disease. Such compositions and methods comprise MASP-2 inhibitors and/or MASP-3 inhibitors and their administration to mammalian subject in need thereof.

The present inventors have discovered that, surprisingly, administration of a MASP-2 inhibitor or a MASP-3 inhibitor in a murine model of SCD results in reduction of SCD disease manifestations such as inflammation, as assessed by levels of liver inflammation biomarkers, and vaso-occlusion.

Accordingly, in one embodiment, the compositions and methods provided herein may be used to treat SCD. In another embodiment, the compositions and methods provided herein may be used to treat, reduce, or prevent vaso-occlusion associated with SCD. In another embodiment, the compositions and method provided herein may be used to treat, reduce, or prevent inflammation associated with SCD. In another embodiment, the compositions and methods provided herein may be used to treat, prevent, and/or reduce the number, duration, and/or severity of crisis episodes associated with SCD.

### III. Overview of the Complement System

The complement system supports innate host defense against pathogens and other acute insults (M.K. Liszewski and J.P. Atkinson, 1993, in Fundamental Immunology, Third Edition, edited by W.E. Paul, Raven Press, Ltd., New York), and also has a role in immune surveillance against cancer (P. Macor, et al., Front. Immunol., 9:2203, 2018). More than 30 fluid-phase and membrane-bound glycoproteins, cofactors, receptors, and regulatory proteins are involved in the complement system (S. Meyer, et al., mAbs, 6:1133, 2014). Many of them are serine proteases, which form a highly regulated cascade of activation events. The complement system responds rapidly to molecular stress signals through a cascade of sequential proteolytic reactions initiated by the binding of pattern recognition receptors (PRRs) to distinct structures on damaged cells, biomaterial surfaces, or microbial intruders (Reis et al., Nat. Rev. Immunol., 18:5, 2018).

Activation of the complement cascade induces diverse immune effector functions, such as cell lysis, phagocytosis, chemotaxis, and immune activation (S. Meyer, et al., 2014). Furthermore, the complement system also acts as a bridge between the innate immune response and the subsequent activation of adaptive immunity. In addition to its anti-infectious properties, the complement system is also involved in the clearance of immune complexes and apoptotic cells, tissue regeneration, mobilization of hematopoietic progenitor cells, and angiogenesis (T.M. Pierpont et al., Front. Oncol., 8:163, 2018).

The complement system can be activated through three distinct pathways: the classical pathway, the alternative pathway, and the lectin pathway. See FIGURE 1. The classical pathway is usually triggered by a complex composed of host antibodies bound to a foreign particle (i.e., an antigen) and thus requires prior exposure to an antigen for the generation of a specific antibody response. Since activation of the classical pathway depends on a prior adaptive immune response by the host, the classical pathway is part of the acquired immune system. In contrast, both the lectin and alternative pathways are independent of adaptive immunity and are part of the innate immune system.

The classical pathway (CP) is initiated by antibody-antigen complexes. Antibodies of subclasses IgM and IgG bind to an antigen on the surface of a pathogen or a target cell and recruit the C1 complex, which is composed of the multimolecular recognition subcomponent C1q (composed of six heterotrimers of the C1q A-chain, B-chain and C-chain) and the C1q-associated serine proteases C1r and C1s. Upon binding of C1q to the Fc-region of either an IgM bound to an antigen or to at least two IgG antibodies bound to their antigens, the serine protease C1r is converted from its zymogen form into its enzymatically active form and cleaves its substrate C1s. Once activated, C1s cleaves C4 into its fragments C4a and C4b. C4b binds to complement component C2 and this complex, C4bC2, is cleaved by C1s in a second cleavage step to release C2a, forming the complement C3 converting enzyme complex C4bC2a, a so called C3 convertase, which cleaves the abundant plasma complement component C3 into C3a and C3b.

The lectin pathway is triggered by the binding of pattern recognition molecules, such as mannose-binding lectin (MBL), ficolins, or collectin-11 and collectin-10, to pathogen-associated molecular patterns (PAMPs) or apoptotic or distressed host cells. The recognition molecules form a complex with the MBL-associated serine proteases, MASP-1 and MASP-2, and activate them upon binding, which results in the cleavage of C2 and C4 and the formation of the C3 convertase (C4bC2a).

The alternative pathway is initiated by spontaneous hydrolysis of C3 ("tickover") to C3(H2O), which binds to factor B (fB). The conversion of the resulting C3(H2O)fB complexes requires enzymatic activity of another highly specific serine protease called factor D. The availability of enzymatically active factor D is thought to be a limiting factor for the alternative pathway amplification loop and the availability of factor D requires the action of another enzyme, MASP-3, which is required for conversion of pro-Factor D (proCFD) into its active form, mature factor D (matCFD) (Dobó et al., 2016). Activated mature factor D (matCFD), another serine protease, cleaves the C3(H2O)-bound fB into Ba and Bb. Bb is also a serine protease, and participates in the formation of alternative C3 pro-convertases C3(H2O)Bb and C3bBb, which cleave C3 into C3a and C3b. The AP amplification loop is formed when freshly generated C3b binds to the target surfaces and sequesters fB to form C3bfB complexes that, upon cleavage by matCFD, create the C3 convertase complex C3bBb. This convertase can be further stabilized by properdin, which prevents decay of the complex and conversion of C3b by factor H and factor I. C3bBb is the functional convertase of the alternative pathway.

The three pathways converge after formation of the C3 convertases C4bC2a and C3bBb. The C3 cleavage fragment C3a is an anaphylatoxin which promotes inflammation. C3b functions as an opsonin by binding covalently through a thioester bond on the surface of target cells, marking them for circulating complement receptor (CR)-displaying effector cells, such as NK cells and macrophages, which contribute to complement-dependent cellular cytotoxicity (CDCC) and complement-dependent cellular phagocytosis (CDCP), respectively. C3b also binds to the C3 convertase (either C2aC4b or C3bBb) to form a C5 convertase (C2aC4b(C3b)n or C3bBb(C3b)n, respectively), which leads to MAC formation and subsequent CDC. Additionally, C3b's cell-bound degradation fragments, iC3b and C3dg, can promote complement-receptor-mediated cytotoxicity (CDCC and CDCP) as well as adaptive immune response through B cell activation (M.C. Carroll, Nat. Immunol., 5:981, 2004). Formation of C5 convertase leads to the cleavage of C5 into C5a and C5b. C5a is another anaphylatoxin. C5b recruits C6-9 to form the membrane attack complex (MAC, or C5b-9 complex). The MAC complex causes pore formation resulting in membrane destruction of the target cell and cell lysis (so called complement-dependent cytotoxicity, CDC). Direct cell lysis through the MAC formation has been traditionally recognized as a terminal effector mechanism of the complement system, however, C3b mediated opsonization and pro-inflammatory signaling as well as the anaphylatoxin function of C3a are thought to play a significant role in the mediation of complement dependent inflammatory pathology.

Complement regulatory proteins (CRPs) prevent unwanted complement activation and consumption of complement components. These proteins are present in most cells and via tight control they play an important role in protecting the host cells from complement-mediated damage. CRPs can be soluble proteins (sCRPs) or membrane-bound complement regulatory proteins (mCRPs) (P.F. Zipfel and C. Skerka, Nat. Rev. Immunol. 9:729, 2009). One of the most abundant protease inhibitors in circulating blood is the C1 inhibitor (C1inh), with an average plasma concentration of 0.25 g/L (H. Gregorek, Comp. and Inflamm. 8:310, 1991). Clinh binds to and inactivates C1r, Cls and two of the MBL-associated serine proteases, MASP-1 and MASP-2; hence it is the primary inhibitor for the classical and lectin pathway. Other sCRPs include C4 binding protein (C4BP), and factors H, B, D, and I (P.F. Zipfel and C. Skerka, 2009).

In contrast to sCRPs, the mCRPs regulate the complement pathways by targeting both C3 and C4 (P.F. Zipfel and C. Skerka, 2009). For example, CD46 (membrane cofactor protein; MCP) is a co-factor for factor I, which mediates cleavage of C3b and C4b into their inactive degradation products, iC3b and iC4b, respectively, and thereby leads to inhibition of all three complement pathways. CD55 (decay acceleration factor; DAF) accelerates the decay of C3 and C5 convertases, which inhibits all three complement pathways. CD59 (protectin) prevents assembly of the MAC by inhibiting the polymerization of C9 and its subsequent binding to C5b-8, thus inhibiting all three pathways.

### IV. MASP-2 and MASP-3 Inhibitors

Provided herein are MASP-2 inhibitors for use in treating, reducing, or preventing sickle cell disease (SCD) and/or various manifestations of SCD. In some embodiments, the MASP-2 inhibitor inhibits lectin pathway-mediated complement activation. Any appropriate MASP-2 inhibitor may be used. In some embodiments, the MASP-2 inhibitor is an antibody or antigen-binding fragment thereof. The antibodies and antigen-binding fragments described herein may be human antibodies, humanized antibodies, chimeric antibodies, murine antibodies, or an antigen-binding fragment of any of the foregoing. Additionally, the antibodies and antigen-binding fragments thereof may be single chain antibodies, ScFv, Fab fragments, Fab' fragments, F(ab')2 fragments, univalent antibodies lacking a hinge region, or whole antibodies. Further, the antibodies and antigen-binding fragments thereof may be monovalent, bivalent, or multivalent. In some embodiments, the antibodies or antigen-binding fragments thereof may comprise a MASP-2 inhibitory peptide.

In certain embodiments, the MASP-2 inhibitor is HG4, which is a fusion protein comprising MASP-2 inhibitory antibody OMS646 and an SGMI-2 peptide that also inhibits MASP-2 activity. As previously described in WO/2017/120344, the SGMI-2 peptide is fused to the C-terminus of the heavy chain of OMS646 to form HG4. The amino acid sequence of the HG4 heavy chain, which comprises a OMS646 heavy chain-SGMI-2 fusion, is provided as SEQ ID NO:1 and the amino acid sequence of the HG4 VL is provided as SEQ ID NO:7.

In some embodiments, the MASP-2 inhibitor is monoclonal antibody OMS646 or an antigen-binding fragment thereof, which may or may not be fused to a MASP-2 inhibitory peptide. In some embodiments, the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10. In some embodiments, the MASP-2 inhibitor is an antibody or antigen-binding fragment thereof comprising a VH comprising SEQ ID NO:3 and a VL comprising SEQ ID NO:7. In some embodiments, the MASP-2 inhibitor comprises a VH comprising a sequence at least 80%, 85%, 90%, 95%, 98%, 99% or 100% identical to SEQ ID NO:3. In some embodiments, the MASP-2 inhibitor comprises a VL comprising a sequence at least 80%, 85%, 90%, 95%, 98%, 99% or 100% identical to SEQ ID NO:7.

In some embodiments, the MASP-2 inhibitor is an antibody or antigen-binding fragment thereof comprising a fusion protein comprising a VH, an IgG constant region, and an inhibitory peptide. In some embodiments, the inhibitory peptide comprises the sequence set forth as SEQ ID NO:2. In some embodiments, the fusion protein comprises the sequence set forth as SEQ ID NO:1. In some embodiments, the fusion protein comprises a sequence at least 80%, 85%, 90%, 95%, 98%, 99% or 100% identical to SEQ ID NO:1.

In some embodiments, the MASP-2 inhibitor is OMS858, which is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26. In some embodiments, the MASP-2 inhibitor is an antibody or antigen-binding fragment thereof comprising a VH comprising SEQ ID NO:19 and a VL comprising SEQ ID NO:23. In some embodiments, the MASP-2 inhibitor comprises a VH comprising a sequence at least 80%, 85%, 90%, 95%, 98%, 99% or 100% identical to SEQ ID NO:19. In some embodiments, the MASP-2 inhibitor comprises a VL comprising a sequence at least 80%, 85%, 90%, 95%, 98%, 99% or 100% identical to SEQ ID NO:23.

In some embodiments, the MASP-2 inhibitor is OMS850, which is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26. In some embodiments, the MASP-2 inhibitor is an antibody or antigen-binding fragment thereof comprising a VH comprising SEQ ID NO:27 and a VL comprising SEQ ID NO:30. In some embodiments, the MASP-2 inhibitor comprises a VH comprising a sequence at least 80%, 85%, 90%, 95%, 98%, 99% or 100% identical to SEQ ID NO:27. In some embodiments, the MASP-2 inhibitor comprises a VL comprising a sequence at least 80%, 85%, 90%, 95%, 98%, 99% or 100% identical to SEQ ID NO:30.

In some embodiments, the MASP-2 inhibitor is OMS852, which is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26. In some embodiments, the MASP-2 inhibitor is an antibody or antigen-binding fragment thereof comprising a VH comprising SEQ ID NO:31 and a VL comprising SEQ ID NO:23. In some embodiments, the MASP-2 inhibitor comprises a VH comprising a sequence at least 80%, 85%, 90%, 95%, 98%, 99% or 100% identical to SEQ ID NO:31. In some embodiments, the MASP-2 inhibitor comprises a VL comprising a sequence at least 80%, 85%, 90%, 95%, 98%, 99% or 100% identical to SEQ ID NO:23.

In some embodiments, the MASP-2 inhibitor is OMS854, which is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26. In some embodiments, the MASP-2 inhibitor is an antibody or antigen-binding fragment thereof comprising a VH comprising SEQ ID NO:32 and a VL comprising SEQ ID NO:23. In some embodiments, the MASP-2 inhibitor comprises a VH comprising a sequence at least 80%, 85%, 90%, 95%, 98%, 99% or 100% identical to SEQ ID NO:32. In some embodiments, the MASP-2 inhibitor comprises a VL comprising a sequence at least 80%, 85%, 90%, 95%, 98%, 99% or 100% identical to SEQ ID NO:23.

In some embodiments, the MASP-2 inhibitor is OMS856, which is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26. In some embodiments, the MASP-2 inhibitor is an antibody or antigen-binding fragment thereof comprising a VH comprising SEQ ID NO:33 and a VL comprising SEQ ID NO:23. In some embodiments, the MASP-2 inhibitor comprises a VH comprising a sequence at least 80%, 85%, 90%, 95%, 98%, 99% or 100% identical to SEQ ID NO:33. In some embodiments, the MASP-2 inhibitor comprises a VL comprising a sequence at least 80%, 85%, 90%, 95%, 98%, 99% or 100% identical to SEQ ID NO:23.

Other MASP-2 inhibitors are known in the art and may alternatively be used. Such MASP-2 inhibitors include the anti-MASP-2 antibodies described in WO 2012/151481, which are hereby incorporated by reference.

Further provided herein are MASP-3 inhibitors for use in treating, reducing, or preventing sickle cell disease (SCD) and/or various manifestations of SCD. In some embodiments, the MASP-3 inhibitor inhibits alternative pathway complement activation. Any appropriate MASP-3 inhibitor may be used. In some embodiments, the MASP-3 inhibitor is an antibody or antigen-binding fragment thereof. The antibodies and antigen-binding fragments described herein may be human antibodies, humanized antibodies, chimeric antibodies, murine antibodies, or an antigen-binding fragment of any of the foregoing. Additionally, the antibodies and antigen-binding fragments thereof may be single chain antibodies, ScFv, Fab fragments, Fab' fragments, F(ab')2 fragments, univalent antibodies lacking a hinge region, or whole antibodies. Further, the antibodies and antigen-binding fragments thereof may be monovalent, bivalent, or multivalent.

In certain embodiments, the MASP-3 inhibitor is MASP-3 inhibitory antibody 13B1, which was previously described in WO 2018/026722. The amino acid sequence of the 13B1 VH is provided as SEQ ID NO:11 and the amino acid sequence of the 13B1 VL is provided as SEQ ID NO:15.

In some embodiments, the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18. In some embodiments, the MASP-3 inhibitor is an antibody or antigen-binding fragment thereof comprising a VH comprising SEQ ID NO:11 and a VL comprising SEQ ID NO:15. In some embodiments, the MASP-3 inhibitor comprises a VH comprising a sequence at least 80%, 85%, 90%, 95%, 98%, 99% or 100% identical to SEQ ID NO:11. In some embodiments, the MASP-3 inhibitor comprises a VL comprising a sequence at least 80%, 85%, 90%, 95%, 98%, 99% or 100% identical to SEQ ID NO:15.

Other MASP-3 inhibitors are known in the art and may alternatively be used. Such MASP-3 inhibitors include the anti-MASP-3 antibodies described in WO 2018/026722, which are hereby incorporated by reference.

The effect of MASP-2 inhibitors and MASP-3 inhibitors on SCD disease manifestations has been demonstrated using a Townes-SS murine mouse model of SCD. For example, SCD patients and Townes-SS mice in vaso-occlusive crisis are known to have elevated alternative pathway Bb fragments in plasma compared to Townes-SS mice not undergoing a vaso-occlusive crisis or wild-type controls. Anti-MASP-3 monoclonal antibody was infused via the tail vein four days prior to a challenge with hypoxia-reoxygenation or hemoglobin infusion. PBS, isotype control antibody, or anti-MASP-2 antibody were infused into Townes-SS mice 30 minutes prior to a challenge with hypoxia-reoxygenation or hemoglobin infusion. In both cases, EDTA plasma was collected four hours after hypoxia-reoxygenation or hemoglobin challenge and analyzed for presence of Bb by immunoblots. Pretreatment with either anti-MASP-2 antibody, a lectin pathway inhibitor, or anti-MASP-3 antibody, an alternative pathway inhibitor, markedly decreased complement Bb fragments in plasma compared to control antibody (see Example 1 and FIGURE 2).

Another SCD disease manifestation, liver inflammation, can induce acute hepatic sequestration or vaso-occlusive crisis (VOC), which has been associated with significant mortality. Since complement activation releases anaphylatoxins C3a and C5a, which are potent inducers of inflammation, markers of inflammation in the livers of SS mice were examined. MASP-2 inhibitor and MASP-3 inhibitor both decreased markers of inflammation in the livers of Townes-SS mice after a challenge with hypoxia-reoxygenation or hemoglobin. Pretreatment with either anti-MASP-2 antibody or anti-MASP-3 antibody markedly decreased NF-κB phospho-P65 expression in hepatic nuclear extracts and VCAM-1, ICAM-1, and E-selectin adhesion molecule expression in hepatic microsomes compared to control antibody (see Example 2 and FIGURE 3).

The ability of MASP-2 inhibitors and MASP-3 inhibitors to decrease microvascular stasis in Townes-SS mice was also investigated. Pretreatment with either anti-MASP-2 antibody or anti-MASP-3 antibody markedly decreased microvascular stasis in Townes-SS mice after a challenge with hypoxia-reoxygenation or hemoglobin compared to Townes-SS mice pretreated with isotype control antibody (see Example 3 and FIGURE 4).

Pain accompanying VOC is a further symptom of SCD that has a significant impact on the quality of life for patients. The ability of MASP-2 inhibitors to reduce or prevent pain by treatment either before the onset of VOC or after onset of VOC was investigated. MASP-2 inhibition was found to result in a reduction of pain from VOC in a treatment model (after onset of VOC), and prevention of pain from VOC in a prevention model (see Examples 4 and 5 and FIGURES 9 and 10).

### V. Pharmaceutical Compositions

Also provided herein are compositions that comprise a MASP-2 inhibitor and/or a MASP-3 inhibitor, and may also include other selected therapeutic agents. Such compositions may further comprise one or more pharmaceutically acceptable carriers, excipients, or diluents. A pharmaceutically acceptable carrier is non-toxic, biocompatible and is selected so as not to detrimentally affect the biological activity of the therapeutic agent (and any other therapeutic agents combined therewith). Examples of pharmaceutically acceptable carriers for peptides are described in U.S. Patent No. 5,211,657 to Yamada. The therapeutic agents described herein may be formulated into preparations in solid, semi solid, gel, liquid, or gaseous forms such as tablets, capsules, powders, granules, ointments, solutions, depositories, inhalants, and injections allowing for oral, parenteral, or surgical administration. Local administration of the compositions by coating medical devices and the like is also contemplated.

Suitable carriers for parenteral delivery via injectable, infusion or irrigation and topical delivery include distilled water, physiological phosphate buffered saline, normal or lactated Ringer's solutions, dextrose solution, Hank's solution, or propanediol. In addition, sterile, fixed oils may be employed as a solvent or suspending medium. For this purpose, any biocompatible oil may be employed including synthetic mono- or diglycerides. In addition, fatty acids such as oleic acid find use in the preparation of injectables. The carrier and agent may be compounded as a liquid, suspension, polymerizable or non-polymerizable gel, paste or salve.

The carrier may also comprise a delivery vehicle to sustain (i.e., extend, delay, or regulate) the delivery of the agent(s) or to enhance the delivery, uptake, stability, or pharmacokinetics of the therapeutic agent(s). Such a delivery vehicle may include, by way of non-limiting example, microparticles, microspheres, nanospheres or nanoparticles composed of proteins, liposomes, carbohydrates, synthetic organic compounds, inorganic compounds, polymeric or copolymeric hydrogels and polymeric micelles. Suitable hydrogel and micelle delivery systems include the PEO:PHB:PEO copolymers and copolymer/cyclodextrin complexes disclosed in WO 2004/009664 A2 and the PEO and PEO/cyclodextrin complexes disclosed in U.S. Patent Application Publication No. 2002/0019369 A1. Such hydrogels may be injected locally at the site of intended action, or subcutaneously or intramuscularly to form a sustained release depot.

Compositions of the present invention may be formulated for delivery by any appropriate method including, without limitation, oral, topical, transdermal, sublingual, buccal, subcutaneously, intra-muscularly, intravenously, intra-arterially or as an inhalant. The compositions of the present invention may also include biocompatible excipients, such as dispersing or wetting agents, suspending agents, diluents, buffers, penetration enhancers, emulsifiers, binders, thickeners, flavoring agents (for oral administration).

Pharmaceutical compositions according to certain embodiments of the present invention are formulated so as to allow the active ingredients contained therein to be bioavailable upon administration of the composition to a patient. Compositions that will be administered to a subject may take the form of one or more dosage units, and a container of a herein described therapeutic agent may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). The composition to be administered will, in any event, contain an effective amount of therapeutic agent or composition of the present disclosure, for treatment of a disease or condition of interest in accordance with teachings herein.

A composition may be in the form of a solid or liquid. In some embodiments, the carrier(s) are particulate, so that the compositions are, for example, in tablet or powder form. The carrier(s) may be liquid, with the compositions being, for example, an oral oil, injectable liquid, or an aerosol, which is useful in, for example, inhalatory administration. When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer, or the like. Such a solid composition will typically contain one or more inert fillers or diluents such as sucrose, corn starch, or cellulose. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin; a flavoring agent such as peppermint, methyl salicylate or orange flavoring; and a coloring agent. When the composition is in the form of a capsule, for example, a gelatin capsule, it may contain, in addition to materials of the above type, a liquid carrier such as polyethylene glycol or oil.

The composition may be in the form of a liquid, for example, an elixir, syrup, solution, emulsion, or suspension. The liquid may be for oral administration or for delivery by injection, as two examples. When intended for oral administration, preferred compositions contain, in addition to the present compounds, one or more of a sweetening agent, preservative, dye/colorant and flavor enhancer. In a composition intended to be administered by injection, one or more of a surfactant, preservative, wetting agent, dispersing agent, suspending agent, buffer, stabilizer, and isotonic agent may be included.

Liquid pharmaceutical compositions, whether they be solutions, suspensions or other like form, may include one or more of the following excipients: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred excipient. An injectable pharmaceutical composition is preferably sterile.

A liquid composition intended for either parenteral or oral administration should contain an amount of a therapeutic agent as described herein such that a suitable dosage will be obtained. The term "parenteral" includes subcutaneous, intravenous, intramuscular, intrasternal, or intraarterial injection or infusion. Typically, the therapeutic agent is at least 0.01% of the composition. When intended for oral administration, this amount may be varied to be between about 0.1% and about 70% of the weight of the composition. Certain oral pharmaceutical compositions contain between about 4% and about 75% therapeutic agent. The composition may be intended for topical administration, in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a composition for topical administration. If intended for transdermal administration, the composition may include a transdermal patch or iontophoresis device. The pharmaceutical composition may be intended for rectal administration, in the form, for example, of a suppository, which will melt in the rectum and release the drug. The composition for rectal administration may contain an oleaginous base as a suitable nonirritating excipient. Such bases include, without limitation, lanolin, cocoa butter, and polyethylene glycol.

A composition may include various materials which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule. The composition in solid or liquid form may include an agent that binds to the therapeutic agent(s) of the disclosure and thereby assists in the delivery of the compound. Suitable agents that may act in this capacity include one or more proteins or a liposome.

The composition may consist essentially of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols may be delivered in single phase, bi-phasic, or tri-phasic system in order to deliver the active ingredient(s). Delivery of the aerosol includes the necessary container, activators, valves, sub-containers, and the like, which together may form a kit. One of ordinary skill in the art, without undue experimentation, may determine preferred aerosols.

The pharmaceutical compositions may be prepared by methodology well known in the pharmaceutical art. For example, a composition intended to be administered by injection can be prepared by combining a composition that comprises therapeutic agent as described herein and optionally, one or more of salts, buffers and/or stabilizers, with sterile, distilled water so as to form a solution. A surfactant may be added to facilitate the formation of a homogeneous solution or suspension. Surfactants are compounds that non-covalently interact with the composition so as to facilitate dissolution or homogeneous suspension in the aqueous delivery system.

### VI. Methods and Uses

Provided herein are methods for the use of MASP-2 inhibitors and/or MASP-3 inhibitors, and compositions comprising the same. In some embodiments, such methods comprise administering one or more MASP-2 inhibitors and/or MASP-3 inhibitors, or a composition comprising the same to a mammalian subject.

Also provided herein are methods of treating sickle cell disease, the methods comprising administering to a mammalian subject in need thereof a therapeutically effective amount of one or more MASP-3 inhibitors, one or more MASP-2 inhibitors; or one or more MASP-3 inhibitors and one or more MASP-2 inhibitors.

Further provided herein are methods of treating, reducing, and/or preventing vaso-occlusion associated with sickle cell disease, the methods comprising administering to a mammalian subject in need thereof a therapeutically effective amount of one or more MASP-3 inhibitors, one or more MASP-2 inhibitors; or one or more MASP-3 inhibitors and one or more MASP-2 inhibitors.

Further provided herein are methods of treating, reducing, and/or preventing inflammation associated with sickle cell disease, the methods comprising administering to a mammalian subject in need thereof a therapeutically effective amount of one or more MASP-3 inhibitors, one or more MASP-2 inhibitors; or one or more MASP-3 inhibitors and one or more MASP-2 inhibitors.

Further provided herein are methods of treating, preventing, and/or reducing the number, duration, and/or severity of crisis episodes associated with sickle cell disease associated with sickle cell disease, the methods comprising administering to a mammalian subject in need thereof a therapeutically effective amount of one or more MASP-3 inhibitors, one or more MASP-2 inhibitors; or one or more MASP-3 inhibitors and one or more MASP-2 inhibitors.

Further provided herein are methods of treating, reducing, and/or preventing pain associated with sickle cell disease, the methods comprising administering to a mammalian subject in need thereof a therapeutically effective amount of one or more MASP-3 inhibitors, one or more MASP-2 inhibitors, or one or more MASP-3 inhibitors and one or more MASP-2 inhibitors.

Further provided herein are methods of treating a mammalian subject suffering from sickle cell disease-related pain, the methods comprising identifying a mammalian subject suffering from pain, diagnosing the pain as relating to sickle cell disease, and administering to the mammalian subject a therapeutically effective amount of one or more MASP-2 inhibitors, one or more MASP-3 inhibitors, or one or more MASP-2 inhibitors and one or more MASP-3 inhibitors, thereby reducing or eliminating the sickle cell disease-related pain in the mammalian subject.

Further provided herein are methods of treating, reducing, and/or preventing acute sickle cell disease symptoms, the methods comprising administering to a mammalian subject in need thereof a therapeutically effective amount of one or more MASP-2 inhibitors, one or more MASP-3 inhibitors, or one or more MASP-2 inhibitors and one or more MASP-3 inhibitors.

Administration of the MASP-2 inhibitors, MASP-3 inhibitors, or compositions of the present disclosure may be by any appropriate route, including oral, topical, transdermal, sublingual, buccal, subcutaneously, intra-muscularly, intravenously, intra-arterially or as an inhalant. The MASP-2 inhibitors and/or MASP-3 inhibitors, or compositions of the present disclosure are administered in a therapeutically effective amount, which amount will vary depending upon a variety of factors including the specific molecules employed, the metabolic stability and length of action of the molecules, the age, sex, body weight, general health, and diet of the subject, the mode and time of administration, the rate of excretion, any additional therapeutic agents administered to the subject in the same time frame, the severity of the particular disorder or disease, and the genetic and epigenetic makeup of the subject. In certain embodiments, the MASP-2 inhibitors, MASP-3 inhibitors, or compositions may be administered to the subject 1, 2, 3, 4, 5, 6, 7, 8, 9, or 10 times, or more. Successive administration may be carried out at any interval, including about 6, about 12, about 24, about 36, about 48, about 74, about 96, or about 108 hours apart, or more.

In some embodiments, the MASP-2 inhibitors, MASP-3 inhibitors, or compositions of the present disclosure are used in combination with other therapeutic agents. Such combination therapy may include administration of a single pharmaceutical dosage formulation that contains MASP-2 inhibitors and/or MASP-3 inhibitors, or compositions comprising the same, together with one or more additional therapeutic agents. Alternatively, such combination therapy may include administration of the MASP-2 inhibitors and/or MASP-3 inhibitors, or compositions comprising the same, and the additional therapeutic agents as separate dosage formulations. Where separate dosage formulations are used, the MASP-2 inhibitors and/or MASP-3 inhibitors, or compositions comprising the same, and the additional therapeutic agents may be administered at essentially the same time, i.e., concurrently, or at separate times, i.e., sequentially in any order. In some embodiments, a combination therapy may comprise administration of a composition comprising two or more different MASP-2 inhibitors and/or MASP-3 inhibitors, or two or more compositions each comprising a MASP-2 inhibitor and/or MASP-3 inhibitor of the present disclosure.

### VII. Sequences

The sequences referred to within the present specification are summarized in Table 1.

**TABLE 1:**

| **SEQ ID NO** | **Description** | **Sequence** |
|---|---|---|
| 1 | HG4 Heavy Chain (OMS646 VH-SGMI-2 fusion, including IgG4 constant region with hinge mutation) (aa) | QVTLKESGPVLVKPTETLTLTCTVSGFSLSRGKMGVS WIRQPPGKALEWLAHIFSSDEKSYRTSLKSRLTISKDT SKNQVVLTMTNMDPVDTATYYCARIRRGGIDYWGQ GTLVTVSSASTKGPSVFPLAPCSRSTSESTAALGCLVK DYFPEPVTVSWNSGALTSGVHTFPAVLQSSGLYSLSS VVTVPSSSLGTKTYTCNVDHKPSNTKVDKRVESKYG PPCPPCPAPEFLGGPSVFLFPPKPKDTLMISRTPEVTCV VVDVSQEDPEVQFNWYVDGVEVHNAKTKPREEQFN STYRVVSVLTVLHQDWLNGKEYKCKVSNKGLPSSIE KTISKAKGQPREPQVYTLPPSQEEMTKNQVSLTCLVK GFYPSDIAVEWESNGQPENNYKTTPPVLDSDGSFFLY SRLTVDKSRWQEGNVFSCSVMHEALHNHYTQKSLSL SLGKAAGGSGLEVTCEPGTTFKDKCNTCRCGSDGKS AVCTKLWCNQGSGA |
| 2 | SGMI-2 (aa) | LEVTCEPGTTFKDKCNTCRCGSDGKSAVCTKLWCNQ |
| 3 | OMS646 VH (aa) | QVTLKESGPVLVKPTETLTLTCTVSGFSLSRGKMGVS WIRQPPGKALEWLAHIFSSDEKSYRTSLKSRLTISKDT SKNQVVLTMTNMDPVDTATYYCARIRRGGIDYWGQ GTLVTVSS |
| 4 | OMS646 HC-CDR1 (aa) | RGKMG |
| 5 | OMS646 HC-CDR2 (aa) | LAHIFSSDEKSYRTSL |
| 6 | OMS646 HC-CDR3 (aa) | YYCARIRRGGIDY |
| 7 | OMS646 VL (aa) | QPVLTQPPSLSVSPGQTASITCSGEKLGDKYAYWYQ QKPGQSPVLVMYQDKQRPSGIPERFSGSNSGNTATLT ISGTQAMDEADYYCQAWDSSTAVFGGGTKLTVL |
| 8 | OMS646 LC-CDR1 (aa) | GEKLGDKYAYW |
| 9 | OMS646 LC-CDR2 (aa) | DKQRPSG |
| 10 | OMS646 LC-CDR3 (aa) | AWDSSTAVF |
| 11 | 13B1 VH (aa) | QVQLKQSGAELMKPGASVKLSCKATGYTFTGKWIE WVKQRPGHGLEWIGEILPGTGSTNYNEKFKGKATFT ADSSSNTAYMQLSSLTTEDSAMYYCLRSEDVWGTGT TVTVSS |
| 12 | 13B1 HC-CDR1 (aa) | GKWIE |
| 13 | 13B1 HC-CDR2 (aa) | EILPGTGSTNYNEKFKG |
| 14 | 13B1 HC-CDR3 (aa) | SEDV |
| 15 | 13B1 VL (aa) | DIVMTQSPSSLAVSAGEKVTMSCKSSQSLLNSRTRKN YLAWYQQKPGQSPKLLIYWASTRESGVPDRFTGSGS GTDFTLTISSVQAEDLAVYYCKQSYNIPTFGGGTKLEI KR |
| 16 | 13B1 LC-CDR1 (aa) | KSSQSLLNSRTRKNYLA |
| 17 | 13B1 LC-CDR2 (aa) | WASTRES |
| 18 | 13B1 LC-CDR3 (aa) | KQSYNIPT |
| 19 | OMS858 VH (aa) | QVQLVQSGAEVKKPGASVKVSCKASGYTFTNHHMH WLRQAPGQGLEWIGDIDASDSETHYIEKFKDRATLTI DKSSSTAYMELSSLRSEDTAVYYCARGDITTTLRYFD VWGQGTLVTVSS |
| 20 | OMS858 HC-CDR1 (aa) | NHHMH |
| 21 | OMS854/856/858 HC-CDR2 (aa) | DIDASDSETHYIEKFKD |
| 22 | OMS850/852/854/8 56/858 HC-CDR3 (aa) | GDITTTLRYFDV |
| 23 | OM852/854/856/85 8 VL (aa) | DIQLTQSPSSLSASVGDRVTITCSASSSVRYMYWYQQ KPGKAPKLLIYDTSNLASGVPSRFSGSGSGTDNTLTIS SLQPEDFATYYCQQWSSYPLTFGQGTKVEIKR |
| 24 | OMS850/852/854/8 56/858 LC-CDR1 (aa) | SASSSVRYMY |
| 25 | OMS850/852/854/8 56/858 LC-CDR2 (aa) | DTSNLAS |
| 26 | OMS850/852/856/8 58/858 LC-CDR3 (aa) | QQWSSYPLT |
| 27 | OMS850 VH (aa) | QVQLQQPGAELVRPGSSVRLSCKASGYTFTNYWMH WLKQRPIQGLEWIGDIDPSDSETHYIEKFKDKATLTID KSSSTAYMHLSSLTSEDSAIYYCARGDITTTLRYFDV WGTGTTVTVSS |
| 28 | OMS850/852/854 HC-CDR1 (aa) | NYWMH |
| 29 | OMS850/852 HC-CDR2 (aa) | DIDPSDSETHYIEKFKD |
| 30 | OMS850 VL (aa) | QIVLTQSPVIMSASPGEKVTMTCSASSSVRYMYWYQ QKPGSSPRLLIYDTSNLASGVPVRFSGSGSGTSNSLTIS RMEAEDAATYYCQQWSSYPLTFGAGTKLELKR |
| 31 | OMS852 VH (aa) | QVQLVQSGAEVKKPGASVKVSCKASGYTFTNYWMH WLRQAPGQGLEWIGDIDPSDSETHYIEKFKDRATLTI DKSSSTAYMELSSLRSEDTAVYYCARGDITTTLRYFD VWGQGTLVTVSS |
| 32 | OMS854 VH (aa) | QVQLVQSGAEVKKPGASVKVSCKASGYTFTNYWMH WLRQAPGQGLEWIGDIDASDSETHYIEKFKDRATLTI DKSSSTAYMELSSLRSEDTAVYYCARGDITTTLRYFD VWGQGTLVTVSS |
| 33 | OMS856 VH (aa) | QVQLVQSGAEVKKPGASVKVSCKASGYTFTNYHMH WLRQAPGQGLEWIGDIDASDSETHYIEKFKDRATLTI DKSSSTAYMELSSLRSEDTAVYYCARGDITTTLRYFD VWGQGTLVTVSS |
| 34 | OMS856 HC-CDR1 (aa) | NYHMH |

### VIII. Examples

### Example 1

### Pre-treatment with MASP-2 Inhibitor or MASP-3 Inhibitor Decreased Complement Factor Bb Fragments in Plasma of SS Mice after Challenge with Hypoxia-Reoxygenation or Hemoglobin.

First, the time courses of inhibition of the alternative pathway (AP) of complement activation by MASP-3 inhibitory antibody 13B1 and of the inhibition of the lectin pathway (LP) of complement activation by MASP-2 inhibitory antibody HG4 in mice were measured. The time course of inhibition of the AP by 13B1 was measured by administering 10 mg/kg of body weight of antibody to C57BL/6 mice by subcutaneous injection. Serum was collected pre-dose and multiple times after antibody administration. AP activity was detected by cytometric detection of C3b/iC3b deposition on zymosan particles. Specifically, zymosan particles (Sigma^{™}) were resuspended to approximately 1 mg/mL in FACs buffer (FB; PBS + 1% BSA) and incubated overnight at 40°C with rotation. The particles were then pelleted and washed two times with GVB buffer (Complement Technology^{™})_by centrifugation at 4000x g at 40°C for five minutes. Zymosan was resuspended to a final concentration of 1 mg/mL in GVB with Mg²⁺ and EGTA (GVB M/E; 10 mM). In a 96-well polypropylene plate, mouse serum was diluted to 5% in GVB M/E (10 mM). 5 µL of the zymosan suspension was added to each well, producing a final reaction volume of 50 µL. The plates were incubated with gentle shaking for 40 minutes at 37°C. The reactions were quenched by three washes in ice-cold FB (centrifugation at 4000x g at 40°C for four minutes). Zymosan pellets were resuspended in 50 µL cold FB with FITC rabbit anti-C3c (1:200; Dako^{™}) and incubated for one hour while shaking at 40°C. The plates were washed three times in cold FB (centrifugation at 4000x g at 40°C for four minutes) and resuspended in 250 µL cold FB for cytometric analysis on a BD FACSCalibur^{™}. Results are shown in FIGURE 6. AP activity in serum was inhibited in a time-dependent fashion, reaching maximal inhibition four days after administration and remaining effective for at least seven days after administration.

The time course of inhibition of the LP by HG4 was measured by administering 1, 5, or 20 mg/kg of HG4 to C57BL/6 mice by subcutaneous injection. Serum samples were collected pre-dose and multiple times after antibody administration. LP activity was assessed in individual serum samples (50% final serum concentration) by quantifying complement C3 deposition on mannan coated ELISA plates. Specifically, serum samples from individual antibody-treated mice diluted to 50% with GVB buffer were added to the wells of ELISA plates coated with 40 µg/mL of mannan and incubated for five minutes at 37°C. To stop lectin pathway activation, the wells were washed three times with ice cold wash buffer (20 mM EDTA, 5% Tween-20 in PBS) followed by two washes with PBS. C3 deposition was detected by incubating ELISA wells with C3c antibody (Dako^{™}) diluted 1:10,000 in PBS containing 0.2% BSA for one hour at room temperature. Following three washes with PBS, wells were incubated with HRP-conjugated goat anti-rabbit IgG (American Qualex Antibodies^{™}) diluted 1:10,000 in PBS containing 0.2% BSA for 1 hour then washed five times with PBS, followed by addition of TMB substrate. Color development was stopped after five minutes and the C3c deposition was quantified by OD450 measurement. LP functional activity was normalized to LP activity in pooled sera from naive C57BL/6 mice. Results are shown in FIGURE 7. Each data point represents mean OD value +/-SEM in serum samples from n=3 mice/group. LP activity was inhibited in a dose-dependent fashion. 5 mg/kg of HG4 maximally inhibited LP activity for at least 48 hours, whereas 20 mg/kg maintained maximal inhibition of LP activity for at least seven days after administration.

To study the effect of MASP-2 or MASP-3 inhibition, Townes-SS mice were used. Townes-SS mice are a murine model of sickle cell disease (SCD). For these studies, equal numbers of male and female Townes-SS mice on a 129/B6 mixed genetic background with knockout of murine α- and β-globin sites and knock-in of human α- and β^{S}-globin into the same genetic sites, as described by Wu et al. (Blood 108:1183 (2006)), were used. Mice were aged 12-35 weeks at the time of the studies.

One group was infused with MASP-3 inhibitor (monoclonal antibody 13B1, 10 mg/kg) via the tail vein four days prior to challenge with hypoxia-reoxygenation (1 h hypoxia at 7% O₂ followed by normoxia for four hours) or hemoglobin infusion (1 µmol/kg). Other groups were infused with isotype control antibody or MASP-2 inhibitor (monoclonal antibody HG4) (each 10 mg/kg) 30 minutes prior to challenge with hypoxia-reoxygenation or hemoglobin infusion. EDTA plasma was collected 4 hours after hypoxia-reoxygenation or hemoglobin challenge and analyzed for Bb expression on immunoblots according to the following procedure.

MASP-2 inhibitor HG4 is a fusion protein comprising MASP-2 inhibitory antibody OMS646 and an SGMI-2 peptide that also inhibits MASP-2 activity. As previously described in WO/2017/120344, the SGMI-2 peptide is fused to the C-terminus of the heavy chain of OMS646 to form HG4. The amino acid sequence of the HG4 heavy chain, which comprises a OMS646 heavy chain-SGMI-2 fusion, is provided as SEQ ID NO:1 and the amino acid sequence of the HG4 VL is provided as SEQ ID NO:7.

MASP-3 inhibitor 13B1 was previously described in WO 2018/026722. The amino acid sequence of the 13B1 VH is provided as SEQ ID NO:11 and the amino acid sequence of the 13B1 VL is provided as SEQ ID NO:15.

EDTA plasma was collected from Townes-SS mice four hours after challenge. Immunoblots of EDTA plasma (5 µL) were immunostained with primary antibodies to complement activation fragment Bb (GeneTex #GTX86947) and to loading control IgG (Bio-Rad #170-6518). Primary antibodies were detected with appropriate secondary antibodies conjugated to alkaline phosphatase and visualized with ECF substrate (GE Healthcare) and a Typhoon FLA 9500 imager (GE Healthcare).

As shown in FIGURE 2, pre-treatment with MASP-2 inhibitor or with MASP-3 inhibitor decreased Bb fragments in plasma of Townes-SS mice after challenge with hypoxia-reoxygenation or hemoglobin infusion.

### Example 2

### Pre-treatment with MASP-2 Inhibitor or MASP-3 Inhibitor Decreased Inflammation Biomarkers in Livers of SS Mice after Challenge with Hypoxia-Reoxygenation or Hemoglobin.

Townes-SS mice were infused with isotype control antibody, MASP-2 inhibitor HG4, or MASP-3 inhibitor 13B1 and challenged with hypoxia-reoxygenation or hemoglobin infusion as described in Example 1.

Livers were collected from the Townes-SS mice four hours after challenge. Microsomes and nuclear extracts were isolated from the liver as previously described (McPherson et al., J Lipid Res 48:86(2007)). Immunoblots of cellular subfractions (30 µg protein) were immunostained with primary antibodies to NF-κB phospho-p65 (Ser536, Cell Signaling #3031), total p65 (Cell Signaling #3034), VCAM-1 (Abcam #174279), and ICAM-1 (Abcam #ab124759), and to loading control GAPDH (Sigma-Aldrich #G9545). NF-κB phospho- and total P65 expression were measured in hepatic nuclear extracts. VCAM-1, ICAM-1, E-selectin, and GAPDH were measured in hepatic microsomes. Primary antibodies were detected with appropriate secondary antibodies conjugated to alkaline phosphatase and visualized with ECF substrate (GE Healthcare) and a Typhoon FLA 9500 imager (GE Healthcare).

As shown in FIGURE 3, pre-treatment with MASP-2 inhibitor or with MASP-3 inhibitor decreased all tested inflammation biomarkers in livers of Townes-SS mice after challenge with hypoxia-reoxygenation or hemoglobin infusion.

### Example 3

### Pre-treatment with MASP-2 Inhibitor or MASP-3 Inhibitor Decreased Microvascular Stasis in SS Mice after Challenge with Hypoxia-Reoxygenation or Hemoglobin.

Townes-SS mice were anesthetized with a mixture of ketamine (106 mg/kg) and xylazine (7.2 mg/kg) and implanted with dorsal skin-fold chambers. After implantation, mice were placed on an intravital microscopy stage and 20-23 flowing subcutaneous venules in the chamber window were selected and mapped as previously described (Belcher, et al., J Mol Med 88:665 (2010)). Mice were infused with MASP-3 inhibitor 13B1 (10 mg/kg body weight) via the tail vein four days prior to challenge with hypoxia-reoxygenation or hemoglobin, or with MASP-2 inhibitor HG4 or isotype control antibody (each 10 mg/kg body weight) 30 minutes prior to challenge with hypoxia-reoxygenation or hemoglobin. Mice challenged with hypoxia-reoxygenation were exposed to hypoxia (7% O₂, 93% N₂) for one hour followed by normoxia for four hours. Mice challenged with hemoglobin were infused via the tail vein with human hemoglobin (1 µmol/kg). Each of the same venules selected and mapped at baseline were visually re-examined for stasis (no flow) at one, two, three, and four hours after hypoxia or one hour after hemoglobin infusion. The static venules in each mouse were counted and percent stasis at one hour was calculated by dividing the number of static venules by the total (static + flowing) number of venules.

As shown in FIGURE 4, pre-treatment with MASP-2 inhibitor or with MASP-3 inhibitor decreased microvascular stasis in Townes-SS mice one hour after challenge with hypoxia-reoxygenation or hemoglobin infusion. Box and whiskers plots in FIGURE 4 represent means ± min to max. Analyses were performed with GraphPad Prism 9.0. ***P<0.001, one-way analysis of variance (ANOVA) (Dunnett's multiple comparisons test).

As shown in FIGURE 5, the effect of pre-treatment with MASP-2 inhibitor or with MASP-3 inhibitor persists through at least four hours after challenge with hypoxia-reoxygenation.

### Example 4

### Pre-treatment with MASP-2 Inhibitor or MASP-3 Inhibitor Reduced Paw Withdrawal Response in SS Mice after Cold Challenge.

The von Frey test is a measure of stimulus-evoked pain used with rodents. The test involves stimulating the plantar surface of a hind paw with a calibrated microfilament and observing whether the animal withdraws the paw in response to the stimulation. Minett et al., Curr Protoc Mouse Biol 1:383 (2011); Deuis et al., Front Mol Neurosci 10:284 (2017). This test was used to measure pain in Townes-SS mice and in Townes-AA mice (humanized with wild-type alpha and beta globin genes) following exposure to cold temperatures, which triggers SCD symptoms in SS mice.

An initial experiment was conducted to demonstrate that cold exposure does trigger a detectable difference in paw withdrawal between Townes-SS mice and Townes-AA mice. Baseline paw withdrawal measurements were made by allowing the mouse to settle on a von Frey rack (an open mesh floor) for 20 minutes, after which a 0.8 mg von Frey monofilament was applied to the plantar surface of a hind paw, applying sufficient pressure to cause the monofilament to bend for three seconds. The monofilament was applied to each of the two hind paws five times, for a total of ten trials, with three seconds between trials. Each mouse was recorded as having withdrawn or not withdrawn the stimulated paw for each trial. For baseline measurements, this process was carried out on two consecutive days. Mice that showed <60% paw withdrawal during these baseline measurements were then subjected to cold exposure within three days. Cold challenge was carried out at 10°C for two hours. The paw withdrawal test was then repeated at 1, 2, 4, and 24hours after completion of the cold challenge. Results are shown in FIGURE 8. Data from five AA and six SS mice is shown. As indicated by the asterisks, all post-cold challenge measurements showed a statistically significant difference in paw withdrawal between the AA and SS mice. For all four of these timepoints, p<0.0001.

The effect of pre-treatment with a MASP-2 or MASP-3 inhibitor on paw withdrawal was then tested. The MASP-2 inhibitor used was anti-MASP-2 monoclonal antibody OMS856. The VH sequence of OMS856 is provided as SEQ ID NO:33 and the VL sequence of OMS856 is provided as SEQ ID NO:23. The MASP-3 inhibitor used was anti-MASP-3 monoclonal antibody 13B1. An unrelated antibody was used as an isotype control. Townes-AA and Townes-SS mice were administered OMS856, 13B1, or control antibody by subcutaneous injection at a dose of 10 mg/kg of body weight four days before cold challenge. Cold challenge was carried out at 10°C for two hours, and paw withdrawal testing was carried out as described above. Withdrawal response was assessed twice at pre-dose (within three days before dosing), once post-dose (three days after dosing), and at 1, 2, 4, 24, and 48 hours post-cold challenge. Results are shown in FIGURE 9. Data from eleven AA mice treated with control antibody is shown along with data from nine SS mice treated with control antibody, twelve SS mice treated with 13B1, and eleven SS mice treated with OMS856. As indicated by the asterisks, all post-cold challenge measurements showed a statistically significant difference in paw withdrawal between the SS mice treated with 13B1 or OMS856 and the SS mice treated with control antibody. At one hour post-challenge, p=0.0134 for 13B1 and p=0.0322 for OMS856. At two hours post-challenge, p=0.0030 for 13B1 and p=0.0233 for OMS856. At four hours post-challenge, p=0.0132 for 13B1 and p=0.0120 for OMS856. At 24 hours post-challenge, p=0.0011 for 13B1 and p=0.0077 for OMS856. At 48 hours post-challenge, p=0.0003 for 13B1 and p=0.0033 for OMS856.

### Example 5

### Treatment with MASP-2 Inhibitor after Cold Challenge

### Reduced Paw Withdrawal Response in SS Mice.

The effect of treatment with a MASP-2 inhibitor on paw withdrawal when the inhibitor is administered after the onset of SCD symptoms was then tested. The MASP-2 inhibitor used was OMS856. Cold challenge was carried out as described in Example 4. Four hours after cold challenge, Townes-AA and Townes-SS mice were administered OMS856 or control antibody by subcutaneous injection at a dose of 10 mg/kg of body weight. Paw withdrawal testing was done at 1, 2, 5, 24, 48, 72, 96, and 168 hours post-cold challenge, using the method described in Example 4. Results are shown in FIGURE 10. Data from four AA mice treated with control antibody is shown along with data from four SS mice treated with control antibody and data from four SS mice treated with OMS856. As indicated by the asterisks, measurements from 48, 72, 96, and 168 hours post-cold challenge showed a statistically significant difference between the SS mice treated with OMS856 and the SS mice treated with control antibody. At 48 hours post-challenge, p=0.012. At 72 hours post-challenge, p=0.0038. At 96 hours post-challenge, p=0.0294. At 168 hours post-challenge, p=0.0136.

### IX. Other Embodiments

All publications, patent applications, and patents mentioned in this specification are herein incorporated by reference.

While certain embodiments of the invention have been illustrated and described, it will be appreciated that various changes can be made therein without departing from the spirit and scope of the invention. Although the invention has been described in connection with specific embodiments, it should be understood that the invention as claimed should not be unduly limited to such specific embodiments. Indeed, various modifications of the specific embodiments described that are obvious to those skilled in the fields of medicine, immunology, pharmacology, or related fields are intended to be within the scope of the invention.

Accordingly, the following numbered paragraphs describing specific embodiments are provided for clarity, but should not be construed to limit the claims.
1. A method of treating sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
2. The method of paragraph 1, wherein the MASP-2 inhibitor inhibits lectin pathway complement activation in the subject.
3. The method of paragraph 1, wherein the MASP-3 inhibitor inhibits alternative pathway complement activation in the subject.
4. The method of paragraph 1 or paragraph 2, wherein the MASP-2 inhibitor is an antibody or antigen-binding fragment thereof.
5. The method of paragraph 1 or paragraph 3, wherein the MASP-3 inhibitor is an antibody or antigen-binding fragment thereof.
6. The method of paragraph 4 or paragraph 5, wherein the antibody or antigen-binding fragment thereof is selected from the group consisting of a human antibody, a humanized antibody, a chimeric antibody, a murine antibody, and an antigen-binding fragment of any of the foregoing.
7. The method of paragraph 4 or paragraph 5, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
8. The method of any of paragraphs 4-7, wherein the antibody or antigen-binding fragment is humanized.
9. The method of any of paragraphs 4-8, wherein the antibody or antigen-binding fragment is fused to a MASP-2 or MASP-3 inhibitory peptide.
10. The method of any of paragraphs 4-9, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
11. The method of paragraph 10, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
12. The method of any of paragraphs 4-9, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO:10.
13. The method of paragraph 12, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
14. The method of paragraph 12, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
15. The method of paragraph 9, wherein the antibody or antigen-binding fragment comprises a fusion protein comprising a VH, an IgG constant region, and an inhibitory peptide.
16. The method of paragraph 15, wherein the inhibitory peptide comprises the sequence set forth as SEQ ID NO:2.
17. The method of paragraph 16, wherein the fusion protein comprises a sequence at least 80%, 85%, 90%, 95%, 98%, 99% or 100% identical to SEQ ID NO:1.
18. The method of any of paragraphs 4-9 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
19. The method of paragraphs 18, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
20. The method of any of paragraphs 4-9 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
21. The method of paragraph 20, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
22. The method of any of paragraphs 4-9 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
23. The method of paragraph 22, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
24. The method of any of paragraph 4-9 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
25. The method of paragraph 24, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
26. The method of any of paragraphs 4-9 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
27. The method of paragraph 26, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
28. A method of treating, reducing, and/or preventing vaso-occlusion associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
29. The method of paragraph 28, wherein the MASP-2 inhibitor inhibits lectin pathway complement activation in the subject.
30. The method of paragraph 28, wherein the MASP-3 inhibitor inhibits alternative pathway complement activation in the subject.
31. The method of paragraph 28 or paragraph 29, wherein the MASP-2 inhibitor is an antibody or antigen-binding fragment thereof.
32. The method of paragraph 28 or paragraph 30, wherein the MASP-3 inhibitor is an antibody or antigen-binding fragment thereof.
33. The method of paragraph 31 or paragraph 32, wherein the antibody or antigen-binding fragment thereof is selected from the group consisting of a human antibody, a humanized antibody, a chimeric antibody, a murine antibody, and an antigen-binding fragment of any of the foregoing.
34. The method of paragraph 31 or paragraph 32, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
35. The method of any of paragraphs 31-34, wherein the antibody or antigen-binding fragment is humanized.
36. The method of any of paragraphs 31-35, wherein the antibody or antigen-binding fragment is fused to a MASP-2 or MASP-3 inhibitory peptide.
37. The method of any of paragraphs 31-36, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
38. The method of paragraph 37, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
39. The method of any of paragraph 31-36, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO:10.
40. The method of paragraph 39, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
41. The method of paragraph 39, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
42. The method of paragraph 36, wherein the antibody or antigen-binding fragment comprises a fusion protein comprising a heavy chain and an inhibitory peptide.
43. The method of paragraph 42, wherein the inhibitory peptide comprises the sequence set forth as SEQ ID NO:2.
44. The method of paragraph 33, wherein the fusion protein comprises a sequence at least 80%, 85%, 90%, 95%, 98%, 99% or 100% identical to SEQ ID NO:1.
45. The method of any of paragraphs 31-36 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
46. The method of paragraph 45, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
47. The method of any of paragraphs 31-36 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
48. The method of paragraph 47, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
49. The method of any of paragraphs 31-36 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
50. The method of paragraph 49, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
51. The method of any of paragraphs 31-36 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
52. The method of paragraph 51, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
53. The method of any of paragraphs 31-36 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
54. The method of paragraph 53, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
55. A method of treating, reducing, and/or preventing inflammation associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
56. The method of paragraph 55, wherein the MASP-2 inhibitor inhibits lectin pathway complement activation in the subject.
57. The method of paragraph 55, wherein the MASP-3 inhibitor inhibits alternative pathway complement activation in the subject.
58. The method of paragraph 55 or paragraph 56, wherein the MASP-2 inhibitor is an antibody or antigen-binding fragment thereof.
59. The method of paragraph 55 or paragraph 57, wherein the MASP-3 inhibitor is an antibody or antigen-binding fragment thereof.
60. The method of paragraph 58 or paragraph 59, wherein the antibody or antigen-binding fragment thereof is selected from the group consisting of a human antibody, a humanized antibody, a chimeric antibody, a murine antibody, and an antigen-binding fragment of any of the foregoing.
61. The method of paragraph 58 or paragraph 59, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
62. The method of any of paragraph 58-61, wherein the antibody or antigen-binding fragment is humanized.
63. The method of any of paragraph 58-61, wherein the antibody or antigen-binding fragment is fused to a MASP-2 or MASP-3 inhibitory peptide.
64. The method of any of paragraphs 58-63, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
65. The method of paragraph 64, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
66. The method of any of paragraphs 58-63, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10.
67. The method of paragraph 66, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
68. The method of paragraph 63, wherein the antibody or antigen-binding fragment comprises a fusion protein comprising a heavy chain and an inhibitory peptide.
69. The method of paragraph 68, wherein the inhibitory peptide comprises the sequence set forth as SEQ ID NO:2.
70. The method of paragraph 69, wherein the fusion protein comprises a sequence at least 80%, 85%, 90%, 95%, 98%, 99% or 100% identical to SEQ ID NO:1.
71. The method of any of paragraphs 58-63 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
72. The method of paragraph 71, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
73. The method of any of paragraphs 58-63 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
74. The method of paragraph 73, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
75. The method of any of paragraphs 58-63 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
76. The method of paragraph 75, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
77. The method of any of paragraphs 58-63 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
78. The method of paragraph 77, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
79. The method of any of paragraphs 58-63 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
80. The method of paragraph 79, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
81. A method of treating, preventing, and/or reducing the number, duration, and/or severity of crisis episodes associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
82. The method of paragraph 81, wherein the MASP-2 inhibitor inhibits lectin pathway complement activation in the subject.
83. The method of paragraph 81, wherein the MASP-3 inhibitor inhibits alternative pathway complement activation in the subject.
84. The method of paragraph 81 or paragraph 82, wherein the MASP-2 inhibitor is an antibody or antigen-binding fragment thereof.
85. The method of paragraph 81 or paragraph 83, wherein the MASP-3 inhibitor is an antibody or antigen-binding fragment thereof.
86. The method of paragraph 84 or paragraph 85, wherein the antibody or antigen-binding fragment thereof is selected from the group consisting of a human antibody, a humanized antibody, a chimeric antibody, a murine antibody, and an antigen-binding fragment of any of the foregoing.
87. The method of paragraph 84 or paragraph 85, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
88. The method of any of paragraphs 84-87, wherein the antibody or antigen-binding fragment is humanized.
89. The method of any of paragraphs 84-88, wherein the antibody or antigen-binding fragment is fused to a MASP-2 or MASP-3 inhibitory peptide.
90. The method of any of paragraphs 84-89, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
91. The method of paragraph 90, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
92. The method of any of paragraphs 84-89, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10.
93. The method of paragraph 92, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
94. The method of paragraph 92, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
95. The method of paragraph 89, wherein the antibody or antigen-binding fragment comprises a fusion protein comprising a heavy chain and an inhibitory peptide.
96. The method of paragraph 95, wherein the inhibitory peptide comprises the sequence set forth as SEQ ID NO:2.
97. The method of paragraph 96, wherein the fusion protein comprises a sequence at least 80%, 85%, 90%, 95%, 98%, 99% or 100% identical to SEQ ID NO:1.
98. The method of any of paragraphs 84-89 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
99. The method of paragraphs 98, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
100. The method of any of paragraphs 84-89 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
101. The method of paragraph 100, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
102. The method of any of paragraphs 84-89 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
103. The method of paragraph 102, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
104. The method of any of paragraphs 84-89 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
105. The method of paragraph 104, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
106. The method of any of paragraphs 84-89 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
107. The method of paragraph 106, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
108. A method of treating, preventing, and/or reducing pain associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
109. The method of paragraph 108, wherein the MASP-2 inhibitor inhibits lectin pathway complement activation in the subject.
110. The method of paragraph 108, wherein the MASP-3 inhibitor inhibits alternative pathway complement activation in the subject.
111. The method of paragraph 108 or paragraph 109, wherein the MASP-2 inhibitor is an antibody or antigen-binding fragment thereof.
112. The method of paragraph 108 or paragraph 110, wherein the MASP-3 inhibitor is an antibody or antigen-binding fragment thereof.
113. The method of paragraph 111 or paragraph 112, wherein the antibody or antigen-binding fragment thereof is selected from the group consisting of a human antibody, a humanized antibody, a chimeric antibody, a murine antibody, and an antigen-binding fragment of any of the foregoing.
114. The method of paragraph 111 or paragraph 112, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
115. The method of any of paragraphs 111-114, wherein the antibody or antigen-binding fragment is humanized.
116. The method of any of paragraphs 111-115, wherein the antibody or antigen-binding fragment is fused to a MASP-2 or MASP-3 inhibitory peptide.
117. The method of any of paragraphs 111-116, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
118. The method of paragraph 117, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
119. The method of any of paragraphs 111-116, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO:10.
120. The method of paragraph 119, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
121. The method of paragraph 119, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
122. The method of paragraph 116, wherein the antibody or antigen-binding fragment comprises a fusion protein comprising a heavy chain and an inhibitory peptide.
123. The method of paragraph 122, wherein the inhibitory peptide comprises the sequence set forth as SEQ ID NO:2.
124. The method of paragraph 123, wherein the fusion protein comprises a sequence at least 80%, 85%, 90%, 95%, 98%, 99% or 100% identical to SEQ ID NO:1.
125. The method of any of paragraphs 111-116 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
126. The method of any of paragraphs 111-116, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
127. The method of any of paragraphs 111-116 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
128. The method of paragraph 127, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
129. The method of any of paragraphs 111-116 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
130. The method of paragraph 129, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
131. The method of any of paragraphs 111-116 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
132. The method of paragraph 131, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
133. The method of any of paragraphs 111-116 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
134. The method of paragraph 133, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
135. A composition comprising a MASP-3 and/or MASP-2 inhibitor for use in treating sickle cell disease.
136. A composition comprising a MASP-3 and/or MASP-2 inhibitor for use in treating, reducing, and/or preventing vaso-occlusion associated with sickle cell disease.
137. A composition comprising a MASP-3 and/or MASP-2 inhibitor for use in treating, reducing, and/or preventing inflammation associated with sickle cell disease.
138. A composition comprising a MASP-3 and/or MASP-2 inhibitor for use in treating, preventing, and/or reducing the number, duration, and/or severity of crisis episodes associated with sickle cell disease.
139. A composition comprising a MASP-3 and/or MASP-2 inhibitor for use in treating, preventing, and/or reducing pain associated with sickle cell disease.
140. The composition of any one of paragraph 135-139, further comprising a pharmaceutically acceptable excipient.
141. The use of a MASP-3 and/or MASP-2 inhibitor for the manufacture of a medicament for the treatment of sickle cell disease; vaso-occlusion associated with sickle cell disease; inflammation associated with sickle cell disease; pain associated with sickle cell disease; or crisis episodes associated with sickle cell disease.
142. A method of treating sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
143. The method of paragraph 142, wherein the MASP-2 inhibitor inhibits lectin pathway complement activation in the subject.
144. The method of paragraph 142, wherein the MASP-3 inhibitor inhibits alternative pathway complement activation in the subject.
145. The method of paragraph 142 or paragraph 144, wherein the MASP-2 inhibitor is an antibody or antigen-binding fragment thereof.
146. The method of paragraph 142 or paragraph 144, wherein the MASP-3 inhibitor is an antibody or antigen-binding fragment thereof.
147. The method of paragraph 145 or paragraph 146, wherein the antibody or antigen-binding fragment thereof is selected from the group consisting of a human antibody, a humanized antibody, a chimeric antibody, a murine antibody, and an antigen-binding fragment of any of the foregoing.
148. The method of paragraph 145 or paragraph 146, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
149. The method of any of paragraphs 145-148, wherein the antibody or antigen-binding fragment is humanized.
150. The method of any of paragraphs 145-149, wherein the antibody or antigen-binding fragment is fused to a MASP-2 or MASP-3 inhibitory peptide.
151. The method of any of paragraphs 146-150, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO: 13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
152. The method of paragraph 151, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
153. The method of any of paragraphs145-150, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10.
154. The method of paragraph 153, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
155. The method of paragraph 153, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
156. The method of paragraph 150, wherein the antibody or antigen-binding fragment comprises a fusion protein comprising a VH, an IgG constant region, and an inhibitory peptide.
157. The method of paragraph 156, wherein the inhibitory peptide comprises the sequence set forth as SEQ ID NO:2.
158. The method of paragraph 157, wherein the fusion protein comprises a sequence at least 80%, 85%, 90%, 95%, 98%, 99% or 100% identical to SEQ ID NO:1.
159. The method of any of paragraphs 145-150 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
160. The method of paragraph 159, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
161. The method of any of paragraphs 145-150 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
162. The method of paragraph 161, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
163. The method of any of paragraphs 145-150 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
164. The method of paragraph 163, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
165. The method of any of paragraphs 145-150 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
166. The method of paragraph 165, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
167. The method of any of paragraphs 145-150 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
168. The method of paragraph 167, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
169. A method of treating, reducing, and/or preventing vaso-occlusion associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
170. The method of paragraph 169, wherein the MASP-2 inhibitor inhibits lectin pathway complement activation in the subject.
171. The method of paragraph 169, wherein the MASP-3 inhibitor inhibits alternative pathway complement activation in the subject.
172. The method of paragraph 169 or paragraph 170, wherein the MASP-2 inhibitor is an antibody or antigen-binding fragment thereof.
173. The method of paragraph 169 or paragraph 171, wherein the MASP-3 inhibitor is an antibody or antigen-binding fragment thereof.
174. The method of paragraph 172 or paragraph 173, wherein the antibody or antigen-binding fragment thereof is selected from the group consisting of a human antibody, a humanized antibody, a chimeric antibody, a murine antibody, and an antigen-binding fragment of any of the foregoing.
175. The method of paragraph 172 or paragraph 173, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
176. The method of any of paragraphs 172-175, wherein the antibody or antigen-binding fragment is humanized.
177. The method of any of paragraphs 172-176, wherein the antibody or antigen-binding fragment is fused to a MASP-2 or MASP-3 inhibitory peptide.
178. The method of any of paragraphs 172-177, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
179. The method of paragraph 178, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
180. The method of any of paragraphs 172-177, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10.
181. The method of paragraph 180, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
182. The method of paragraph 180, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
183. The method of paragraph 177, wherein the antibody or antigen-binding fragment comprises a fusion protein comprising a heavy chain and an inhibitory peptide.
184. The method of paragraph 183, wherein the inhibitory peptide comprises the sequence set forth as SEQ ID NO:2.
185. The method of paragraph 174, wherein the fusion protein comprises a sequence at least 80%, 85%, 90%, 95%, 98%, 99% or 100% identical to SEQ ID NO:1.
186. The method of any of paragraphs 172-177 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
187. The method of paragraph 186, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
188. The method of any of paragraphs 172-177 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
189. The method of paragraph 188, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
190. The method of any of paragraphs 172-177 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
191. The method of paragraph 190, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
192. The method of any of paragraphs 172-177 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
193. The method of paragraph 192, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
194. The method of any of paragraphs 172-177 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
195. The method of paragraph 194, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
196. A method of treating, reducing, and/or preventing inflammation associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
197. The method of paragraph 196, wherein the MASP-2 inhibitor inhibits lectin pathway complement activation in the subject.
198. The method of paragraph 196, wherein the MASP-3 inhibitor inhibits alternative pathway complement activation in the subject.
199. The method of paragraph 196 or paragraph 197, wherein the MASP-2 inhibitor is an antibody or antigen-binding fragment thereof.
200. The method of paragraph 196 or paragraph 198, wherein the MASP-3 inhibitor is an antibody or antigen-binding fragment thereof.
201. The method of paragraph 199 or paragraph 200, wherein the antibody or antigen-binding fragment thereof is selected from the group consisting of a human antibody, a humanized antibody, a chimeric antibody, a murine antibody, and an antigen-binding fragment of any of the foregoing.
202. The method of paragraph 199 or paragraph 200, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
203. The method of any of paragraphs 199-202, wherein the antibody or antigen-binding fragment is humanized.
204. The method of any of paragraphs 199-202, wherein the antibody or antigen-binding fragment is fused to a MASP-2 or MASP-3 inhibitory peptide.
205. The method of any of paragraphs 199-204, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
206. The method of paragraph 205, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
207. The method of any of paragraphs 109-204, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO:10.
208. The method of paragraph 207, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
209. The method of paragraph 204, wherein the antibody or antigen-binding fragment comprises a fusion protein comprising a heavy chain and an inhibitory peptide.
210. The method of paragraph 209, wherein the inhibitory peptide comprises the sequence set forth as SEQ ID NO:2.
211. The method of paragraph 210, wherein the fusion protein comprises a sequence at least 80%, 85%, 90%, 95%, 98%, 99% or 100% identical to SEQ ID NO:1.
212. The method of any of paragraphs 199-204 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
213. The method of paragraph 212, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
214. The method of any of paragraphs 199-204 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
215. The method of paragraph 214, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
216. The method of any of paragraphs 199-204 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
217. The method of paragraph 216, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
218. The method of any of paragraphs 199-204 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
219. The method of paragraph 218, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
220. The method of any of paragraphs 199-204 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
221. The method of paragraph 220, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
222. A method of treating, preventing, and/or reducing the number, duration, and/or severity of crisis episodes associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
223. The method of paragraph 222, wherein the MASP-2 inhibitor inhibits lectin pathway complement activation in the subject.
224. The method of paragraph 222, wherein the MASP-3 inhibitor inhibits alternative pathway complement activation in the subject.
225. The method of paragraph 222 or paragraph 223, wherein the MASP-2 inhibitor is an antibody or antigen-binding fragment thereof.
226. The method of paragraph 222 or paragraph 224, wherein the MASP-3 inhibitor is an antibody or antigen-binding fragment thereof.
227. The method of paragraph 225 or paragraph 226, wherein the antibody or antigen-binding fragment thereof is selected from the group consisting of a human antibody, a humanized antibody, a chimeric antibody, a murine antibody, and an antigen-binding fragment of any of the foregoing.
228. The method of paragraph 225 or paragraph 226, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
229. The method of any of paragraphs 225-228, wherein the antibody or antigen-binding fragment is humanized.
230. The method of any of paragraphs 225-229, wherein the antibody or antigen-binding fragment is fused to a MASP-2 or MASP-3 inhibitory peptide.
231. The method of any of paragraphs 225-230, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
232. The method of paragraph 231, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
233. The method of any of paragraphs 225-230, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10.
234. The method of paragraph 233, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
235. The method of paragraph 233, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
236. The method of paragraph 230, wherein the antibody or antigen-binding fragment comprises a fusion protein comprising a heavy chain and an inhibitory peptide.
237. The method of paragraph 236, wherein the inhibitory peptide comprises the sequence set forth as SEQ ID NO:2.
238. The method of paragraph 237, wherein the fusion protein comprises a sequence at least 80%, 85%, 90%, 95%, 98%, 99% or 100% identical to SEQ ID NO:1.
239. The method of any of paragraphs 225-230 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
240. The method of paragraph 239, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
241. The method of any of paragraphs 225-230 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
242. The method of paragraph 241, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
243. The method of any of paragraphs 225-230 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
244. The method of paragraph 243, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
245. The method of any of paragraphs 225-230 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
246. The method of paragraph 245, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
247. The method of any of paragraphs 225-230 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
248. The method of paragraph 247, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
249. A method of treating, preventing, and/or reducing pain associated with sickle cell disease, the method comprising administering to a mammalian subject in need thereof a therapeutically effective amount of:
   i. a MASP-2 inhibitor;
   ii. a MASP-3 inhibitor; or
   iii. a MASP-2 inhibitor and a MASP-3 inhibitor.
250. The method of paragraph 249, wherein the MASP-2 inhibitor inhibits lectin pathway complement activation in the subject.
251. The method of paragraph 249, wherein the MASP-3 inhibitor inhibits alternative pathway complement activation in the subject.
252. The method of paragraph 249 or paragraph 250, wherein the MASP-2 inhibitor is an antibody or antigen-binding fragment thereof.
253. The method of paragraph 249 or paragraph 251, wherein the MASP-3 inhibitor is an antibody or antigen-binding fragment thereof.
254. The method of paragraph 252 or paragraph 253, wherein the antibody or antigen-binding fragment thereof is selected from the group consisting of a human antibody, a humanized antibody, a chimeric antibody, a murine antibody, and an antigen-binding fragment of any of the foregoing.
255. The method of paragraph 252 or paragraph 253, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.
256. The method of any of paragraphs 252-255, wherein the antibody or antigen-binding fragment is humanized.
257. The method of any of paragraphs 252-256, wherein the antibody or antigen-binding fragment is fused to a MASP-2 or MASP-3 inhibitory peptide.
258. The method of any of paragraphs 252-257, wherein the MASP-3 inhibitor is an antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO:18.
259. The method of paragraphs 258, wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.
260. The method of any of paragraphs 252-257, wherein the MASP-2 inhibitor is an antibody or antigen-binding fragment thereof comprising a heavy chain variable region comprising a HC-CDR1 set forth as SEQ ID NO:4, a HC-CDR2 set forth as SEQ ID NO:5, and a HC-CDR3 set forth as SEQ ID NO:6; and a light chain variable region comprising a LC-CDR1 set forth as SEQ ID NO:8, a LC-CDR2 set forth as SEQ ID NO:9, and a LC-CDR3 set forth as SEQ ID NO: 10.
261. The method of paragraph 260, wherein the antibody or antigen-binding fragment thereof comprises a VH comprising a sequence at least 80%, 85%, 90%, 95%, 98%, 99% or 100% identical to SEQ ID NO:3 and a VL comprising a sequence at least 80%, 85%, 90%, 95%, 98%, 99% or 100% identical to SEQ ID NO:7.
262. The method of paragraph 260, wherein the antibody or antigen-binding fragment thereof comprises a heavy chain comprising a sequence at least 80%, 85%, 90%, 95%, 98%, 99% or 100% identical to SEQ ID NO:1 and a VL comprising a sequence at least 80%, 85%, 90%, 95%, 98%, 99% or 100% identical to SEQ ID NO:7.
263. The method of paragraph 257, wherein the antibody or antigen-binding fragment comprises a fusion protein comprising a heavy chain and an inhibitory peptide.
264. The method of paragraph 263, wherein the inhibitory peptide comprises the sequence set forth as SEQ ID NO:2.
265. The method of paragraph 264, wherein the fusion protein comprises a sequence at least 80%, 85%, 90%, 95%, 98%, 99% or 100% identical to SEQ ID NO:1.
266. The method of any of paragraphs 252-257 wherein the MASP-2 inhibitor is an antibody or antigen-binding fragment thereof comprising a heavy chain variable region comprising a HC-CDR1 set forth as SEQ ID NO:34, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
267. The method of any of paragraphs 252-257, wherein the antibody or antigen-binding fragment thereof comprises a heavy chain comprising a sequence at least 80%, 85%, 90%, 95%, 98%, 99% or 100% identical to SEQ ID NO:33 and a VL comprising a sequence at least 80%, 85%, 90%, 95%, 98%, 99% or 100% identical to SEQ ID NO:23.
268. The method of any of paragraphs 252-257 wherein the MASP-2 inhibitor is an antibody or antigen-binding fragment thereof comprising a heavy chain variable region comprising a HC-CDR1 set forth as SEQ ID NO:20, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
269. The method of paragraph 268, wherein the antibody or antigen-binding fragment thereof comprises a heavy chain comprising a sequence at least 80%, 85%, 90%, 95%, 98%, 99% or 100% identical to SEQ ID NO:19 and a VL comprising a sequence at least 80%, 85%, 90%, 95%, 98%, 99% or 100% identical to SEQ ID NO:23.
270. The method of any of paragraphs 252-257 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
271. The method of paragraph 270, wherein the antibody or antigen-binding fragment thereof comprises a heavy chain comprising a sequence at least 80%, 85%, 90%, 95%, 98%, 99% or 100% identical to SEQ ID NO:27 and a VL comprising a sequence at least 80%, 85%, 90%, 95%, 98%, 99% or 100% identical to SEQ ID NO:30.
272. The method of any of paragraphs 252-257 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:29, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
273. The method of paragraph 272, wherein the antibody or antigen-binding fragment thereof comprises a heavy chain comprising a sequence at least 80%, 85%, 90%, 95%, 98%, 99% or 100% identical to SEQ ID NO:31 and a VL comprising a sequence at least 80%, 85%, 90%, 95%, 98%, 99% or 100% identical to SEQ ID NO:23.
274. The method of any of paragraphs 252-257 wherein the MASP-2 inhibitor is an antibody or antigen-binding fragment thereof comprising a heavy chain variable region comprising a HC-CDR1 set forth as SEQ ID NO:28, a HC-CDR2 set forth as SEQ ID NO:21, and a HC-CDR3 set forth as SEQ ID NO:22; and a light chain variable region comprising a LC-CDR1 set forth as SEQ ID NO:24, a LC-CDR2 set forth as SEQ ID NO:25, and a LC-CDR3 set forth as SEQ ID NO:26.
275. The method of paragraph 274, wherein the antibody or antigen-binding fragment thereof comprises a heavy chain comprising a sequence at least 80%, 85%, 90%, 95%, 98%, 99% or 100% identical to SEQ ID NO:32 and a VL comprising a sequence at least 80%, 85%, 90%, 95%, 98%, 99% or 100% identical to SEQ ID NO:23.
276. A composition comprising a MASP-3 and/or MASP-2 inhibitor for use in treating sickle cell disease.
277. A composition comprising a MASP-3 and/or MASP-2 inhibitor for use in treating, reducing, and/or preventing vaso-occlusion associated with sickle cell disease.
278. A composition comprising a MASP-3 and/or MASP-2 inhibitor for use in treating, reducing, and/or preventing inflammation associated with sickle cell disease.
279. A composition comprising a MASP-3 and/or MASP-2 inhibitor for use in treating, preventing, and/or reducing the number, duration, and/or severity of crisis episodes associated with sickle cell disease.
280. A composition comprising a MASP-3 and/or MASP-2 inhibitor for use in treating, preventing, and/or reducing pain associated with sickle cell disease.
281. The composition of any one of paragraphs 276-280, further comprising a pharmaceutically acceptable excipient.
282. The use of a MASP-3 and/or MASP-2 inhibitor for the manufacture of a medicament for the treatment of sickle cell disease; vaso-occlusion associated with sickle cell disease; inflammation associated with sickle cell disease; pain associated with sickle cell disease; or crisis episodes associated with sickle cell disease.

## Claims

1. A composition comprising a MASP-3 inhibitory antibody for use in treating sickle cell disease.

2. The composition for use according to claim 1, wherein the MASP-3 inhibitory antibody inhibits alternative pathway complement activation in the subject.

3. The composition for use according to claim 1 or claim 2, wherein the antibody or antigen-binding fragment thereof is selected from the group consisting of a human antibody, a humanized antibody, a chimeric antibody, a murine antibody, and an antigen-binding fragment of any of the foregoing.

4. The composition for use according to any of claims 1-3, wherein the antibody or antigen-binding fragment thereof is selected from the group consisting of a single chain antibody, an ScFv, a Fab fragment, an Fab' fragment, an F(ab')2 fragment, a univalent antibody lacking a hinge region and a whole antibody.

5. The composition for use according to claim 3, wherein the antibody or antigen-binding fragment is humanized.

6. The composition for use according to any of claims 1-5, wherein the antibody or antigen-binding fragment is fused to a MASP-3 inhibitory peptide.

7. The composition for use according to any of claims 1-6, wherein the MASP-3 inhibitory antibody or antigen-binding fragment thereof comprising a heavy chain variable region comprising a HC-CDR1 set forth as SEQ ID NO:12, a HC-CDR2 set forth as SEQ ID NO:13, and a HC-CDR3 set forth as SEQ ID NO:14; and a light chain variable region comprising a LC-CDR1 set forth as SEQ ID NO:16, a LC-CDR2 set forth as SEQ ID NO:17, and a LC-CDR3 set forth as SEQ ID NO: 18; and optionally wherein the antibody or antigen-binding fragment thereof comprises a VH comprising a sequence at least 80%, 85%, 90%, 95%, 98%, 99% or 100% identical to SEQ ID NO:11 and a VL comprising a sequence at least 80%, 85%, 90%, 95%, 98%, 99% or 100% identical to SEQ ID NO:15.

8. A composition comprising a MASP-3 inhibitory antibody for use in treating, reducing, and/or preventing vaso-occlusion associated with sickle cell disease; inflammation associated with sickle cell disease; pain associated with sickle cell disease; or for use in treating, preventing, and/or reducing the number, duration, and/or severity of crisis episodes associated with sickle cell disease.

9. The composition for use according to any one of claims 1-8, further comprising a pharmaceutically acceptable excipient.
